# EUROPEAN PATENT SPECIFICATION

(11) **EP 3 904 498 B1**
(45) Date of publication and mention of the grant of the patent: **21.12.2022**
(21) Application number: 20749727.2
(22) Date of filing: 07.01.2020
(51) Int. Cl.: B06B 1/02, C12N 15/10, G01N 1/28, A61B 10/02

(54) **HANDHELD ANIMAL AND PLANT TISSUE ABLATOGRAPH BASED ON THE PRINCIPLE OF ULTRASOUND**
HANDGEHALTENER TIER- UND PFLANZENGEWEBEABLATOGRAF AUF BASIS DES ULTRASCHALLPRINZIPS
ABLATOGRAPHE PORTATIF POUR TISSU ANIMAL ET VÉGÉTAL BASÉ SUR LE PRINCIPE DES ULTRASONS

(30) Priority: 01.02.2019 CN 201910102427
(43) Date of publication of application: 03.11.2021
(73) Proprietor: Chengdu Daosheng Biotechnology, Co., Ltd., Chengdu, Sichuan 610000 (CN)
(72) Inventor: ZHAO, Zhongjiu, Chengdu, Sichuan 610000 (CN); ZHAO, Wei, Chengdu, Sichuan 610000 (CN); LIAO, Dongsheng, Chengdu, Sichuan 610000 (CN); QIU, Kun, Panzhihua, Sichuan 617000 (CN)
(74) Representative: Petculescu, Ana-Maria
(86) International application number: PCT/CN2020/070558
(87) International publication number: WO 2020/156047

(56) References cited:
- EP-A1- 3 243 467
- WO-A1-02/094103
- CN-A- 101 658 838
- CN-A- 103 252 314
- CN-A- 106 139 426
- CN-A- 107 920 850
- CN-A- 109 652 312
- CN-A- 109 694 826
- CN-U- 209 508 290
- CN-U- 209 584 276
- US-A1- 2004 073 209
- GONG JING-SHUANG ET AL: "A design of intelligent power ultrasonic supply", 2016 SYMPOSIUM ON PIEZOELECTRICITY, ACOUSTIC WAVES, AND DEVICE APPLICATIONS (SPAWDA), IEEE, 21 October 2016 (2016-10-21), pages 293-296, XP033051885, DOI: 10.1109/SPAWDA.2016.7830008 [retrieved on 2017-01-23]
- Peng Huanrong , Huang Yong-feng: "Control Frequency-Tracking of Ultrasonic Transducer with Single-Chip Microcomputer", China Measurement & Testing Technology, vol. 34, no. 4, 31 July 2008 (2008-07-31), pages 106-108, XP009522479, ISSN: 1674-5124
- Guo, Haiyan: "The Application of MPU on the Energy Converter of the Piezoelectric Ceramic Ultrasonic Waves", Journal of Zhangzhou Teachers College (Natural Science Edition), vol. 16, no. 1, 28 February 2003 (2003-02-28), pages 58-61, XP009522481, CN ISSN: 2095-7122, DOI: 10.16007/j.cnki.issn2095-7122.2003.01.013

## Description

### TECHNICAL FIELD

The present invention relates to the technical field of RNA, DNA or protein extraction, and more particularly, to an ultrasound-based handheld animal and plant tissue ablation instrument.

### BACKGROUND

An ultrasonic vibrator is also called an ultrasonic oscillator. The ultrasonic vibrator can realize mutual conversion between electrical energy and mechanical energy (acoustic vibration) by piezoelectric effect of piezoelectric ceramics and performs amplification by front and rear radiation cover blocks matched with the acoustic impedance. The ultrasonic vibrator is an ultrasonic vibration system formed by an ultrasonic transducer and an ultrasonic amplitude transformer. The ultrasonic transducer can convert high-frequency electrical energy into mechanical energy. The ultrasonic amplitude transformer as a passive device does not generate vibration, but only transmits out the vibration input by the ultrasonic transducer after changing its amplitude, so that impedance transformation is completed. The ultrasonic transducer can produce regular vibration under appropriate electric field excitation, with an amplitude of generally about 10 µm. Such an amplitude is not enough for direct welding and processing. To solve this, the transducer is connected to a reasonably designed ultrasonic amplitude transformer, so that the amplitude of the ultrasonic vibration can vary in a wide range. As long as the strength of materials is enough, the amplitude can exceed 100 µm. When the ultrasonic amplitude transformer performs vibration of longitudinal expansion and contraction, movement directions of the mass points on the left and right sides of a cross section are opposite, which is equivalent to the existence of a relatively static nodal surface. This nodal surface is called a node, which is also the best fixed point for the vibrator. Deviation from this node will reduce the working efficiency of the vibrator, which is commonly known as leaky wave.

Common applications of ultrasonic vibrators include: ultrasonic cleaners, ultrasonic atomizers, B-ultrasound probes, and others. The prior ultrasonic vibrators, however, have the problems of inconvenience in installing the structure and inconvenience in holding the finished structure after installation.

As can be seen from the above, there is no related application of the ultrasonic vibrator in the prior methods for rapidly extracting RNA, DNA or protein from tissues. To rapidly extract RNA, DNA or protein from tissues, a tissue grinder produced by Thermo Fisher Scientific from the USA grinds tissues into the tissue homogenate through irregular high-speed movement of magnetic beads in the electromagnetic field, but the machine is expensive and can only homogenize the tissues into relatively small tissue masses. The handheld/desktop dual-use homogenizer PR0^{∗}PR0200 and the high-throughput biological sample homogenizer Bioprep-24 from the USA are also expensive, and can only homogenize the tissues into relatively small tissue masses. EP3243467A1 discloses an ultrasonic treatment instrument for use in arthroscopic surgery, wherein the instrument includes: a probe having a main body that is configured to transmit ultrasonic vibration, and a treatment portion that is provided on a distal side of the main body and is configured to cut a hard tissue and/or a soft tissue by transmission of the ultrasonic vibration; and a sheath unit that includes an inner sheath covering an outer peripheral surface of the main body in the probe, and an outer sheath covering an outer peripheral surface of the inner sheath and forming a suction path between the outer peripheral surface of the inner sheath and the outer sheath, wherein a distal end of the inner sheath extends to a position closer to the treatment portion of the probe than a distal end of the outer sheath. CN103252314A discloses a dynamic matching device of an ultrasonic power supply and a method thereof, wherein the dynamic matching device comprises an ultrasonic signal generator, a driving isolating circuit, an ultrasonic power amplifier, a matching transformer, a tuning inductor and an ultrasonic transducer which are successively in serial connection, and further comprises a voltage and current sampling circuit, wherein the voltage and current sampling circuit is successively connected through a backward stage isolating circuit, a filter shaping circuit and a programmable logic device (PLD), a phase measurement unit is arranged in the PLD, and a pulse width modulation (PWM) drive pulse output end of the PLD is connected with the ultrasonic signal generator. According to the dynamic matching device and the method thereof, frequency of ultrasonic driving signals is dynamically regulated through real-time monitoring of the impedance characteristic of an ultrasonic transducer circuit, a dynamic matching process is achieved.

### SUMMARY

In order to resolve the above problems in the prior art, the present invention provides an ultrasound-based handheld animal and plant tissue ablation instrument.

The technical solutions adopted by the present invention are as follows:

An ultrasound-based handheld animal and plant tissue ablation instrument includes a housing internally provided with a control board and a drive board, and an ultrasonic vibrator fixedly connected to a front end of the housing;
the ultrasonic vibrator includes an ultrasonic transducer and an amplitude transformer; the amplitude transformer is fixedly connected to a front end of the ultrasonic transducer, is configured to change an amplitude of vibration input by the ultrasonic transducer and to transmit out the vibration;
the control board is provided with a power supply, a direct current to direct current (DCDC) conversion unit, a main control unit, a DCDC power adjustment unit and a sampling unit; the drive board is provided with a drive unit, a transformer unit and a resonance unit; a voltage of the power supply is converted through the DCDC conversion unit and the DCDC power adjustment unit and then is supplied to the animal and plant tissue ablation instrument; the main control unit outputs a first pulse width modulation (PWM) signal to control the DCDC power adjustment unit to output an adjustable voltage, and outputs two second PWM signals with complementary duty cycles to the drive unit based on feedback from the sampling unit that acquires the voltage and the current of an output circuit of the transformer unit, the transformer unit transforms the voltage, and then the resonance unit arranged in the output circuit of the transformer unit adjusts a resonance point to a resonance point of the ultrasonic vibrator, so that the ultrasonic vibrator arranged in the output circuit of the transformer unit operates in a resonance state; and
the housing is a handheld housing, the housing includes a first section and a second section that are disposed at an obtuse angle to each other, and an end of the first section away from the second section is the front end of the housing.

On the basis of the above technical solutions, preferably, the front end of the housing is provided with a connecting hole for fixedly connecting to the ultrasonic vibrator, an inner wall of the connecting hole is provided with one or more first clamping slots, and correspondingly, the ultrasonic vibrator is provided with a first step matched with the first clamping slot.

Preferably, the ultrasonic vibrator is installed at the front end of the housing through snap-fitting, so that the ultrasonic vibrator is stable in installation and convenient for maintenance and disassembly.

On the basis of the above technical solutions, preferably, both sides of the first step are provided with a first clamping block and a second clamping block respectively, and the first step is clamped in the first clamping slot through the first clamping block and the second clamping block.

Preferably, the first clamping block and the second clamping block help to clamp the ultrasonic vibrator, to stably install the ultrasonic vibrator further.

On the basis of the above technical solutions, preferably, the first clamping slot is a circular clamping slot, the first step is a circular step, both the first clamping block and the second clamping block are circular clamping blocks, and the first clamping block and the second clamping block are both sleeved on an outer wall of the ultrasonic vibrator.

On the basis of the above technical solutions, preferably, a front end of the ultrasonic vibrator provided with the first step is further sleeved with a first fixed cover, and a rear end of the first fixed cover is clamped in the first clamping slot.

Preferably, the first fixed cover extends to fix a middle and rear end of the ultrasonic vibrator. In this way, the installation stability of the ultrasonic vibrator is increased, and the ultrasonic vibrator achieves a good effect. In addition, the service life of the ultrasonic vibrator is increased.

On the basis of the above technical solutions, preferably, the housing includes an upper housing and a lower housing that are engaged with each other; a front end of the upper housing and a front end of the lower housing are further sleeved with a second fixed cover, an inner wall of the second fixed cover is provided with a second step, and outer walls of the front end of the upper housing and the front end of the lower housing are further provided with a second clamping slot matched with the second step.

The installation method of the upper housing and the lower housing facilitates the installation, maintenance and disassembly of the housing, and further secures the ultrasonic vibrator.

On the basis of the above technical solutions, preferably, a hook is arranged on an outer wall of the housing, which is convenient for placing the present invention.

On the basis of the above technical solutions, preferably, the animal and plant tissue ablation instrument has power of 0.5-1000 W, and ultrasound frequency of 20-200 kHz.

On the basis of the above technical solutions, preferably, the DCDC power adjustment unit includes a switch control signal receiving circuit whose switch control signal receiving terminal is connected to the main control unit to receive a switch control signal;
a first PWM signal receiving circuit whose first PWM signal receiving terminal is connected to the main control unit to receive the first PWM signal; and
a fourth DCDC conversion unit configured to convert, through the switch control signal and the first PWM signal sent by the main control unit, the power supply to the adjustable voltage for output.

On the basis of the above technical solutions, preferably, the fourth DCDC conversion unit includes a fourth voltage input filter, a fourth voltage conversion chip U1 and a fourth voltage output filter that sequentially process the voltage of the power supply; the fourth voltage input filter includes a filter capacitor connected to the power supply; an upper tube drive signal reference point pin SW of the fourth voltage conversion chip U1 is connected to an adjustable voltage output terminal through an energy storage inductor L1; voltage divider resistors R32 and R47 connected in series with the adjustable voltage output terminal sample a voltage and input the voltage to a reference voltage pin FB of the fourth voltage conversion chip U1; the upper tube drive signal reference point pin SW of the fourth voltage conversion chip U1 is further connected to a freewheeling diode D2; an enable pin EN is connected to a resistor R33; a resistor timing/external clock pin RT/CLK is connected to a frequency divider resistor R85; a frequency compensation pin COMP is connected to a capacitor C51, a capacitor C49 and a resistor R46 that are used to adjust a circuit and stabilize voltage output, where the capacitor C51 and the resistor R46 are connected in series and then are connected with the capacitor C49 in parallel; the fourth voltage output filter includes a filter capacitor connected to the adjustable voltage output terminal;
the switch control signal receiving circuit includes a resistor R104, a resistor R105 and a transistor Q12, wherein the resistor R104 and the resistor R105 are connected in series between the converted voltage and the switch control signal receiving terminal, an emitter of the transistor Q12 is connected to the converted voltage, a base of the transistor Q12 is connected to a node between the resistor R104 and the resistor R105, and a collector of the transistor Q12 is connected to the enable pin EN of the fourth voltage conversion chip U1; when the switch control signal is L, the transistor Q12 is turned on, and the fourth voltage conversion chip U1 is activated to work; and
the first PWM signal receiving circuit includes an RC filter and a first voltage follower U3B that sequentially process the first PWM signal; the first PWM signal received by the first PWM signal receiving terminal is filtered through the RC filter and then input to a non-inverting input terminal of the first voltage follower U3B, and an output terminal of the first voltage follower U3B is connected to a node between the voltage divider resistors R32 and R47 through a voltage divider resistor R54.

On the basis of the above technical solutions,_preferably, the drive unit includes a first drive unit and a second drive unit; a second PWM signal receiving terminal N of the first drive unit receives a first-channel second PWM signal sent by the main control unit, and an output terminal of the first drive unit is connected to a dotted terminal of a primary coil of the transformer unit; a second PWM signal receiving terminal P of the second drive unit receives a second-channel second PWM signal sent by the main control unit, and an output terminal of the second drive unit is connected to a non-dotted terminal of the primary coil of the transformer unit; the first-channel second PWM signal received by the second PWM signal receiving terminal N controls a first drive metal-oxide-semiconductor (MOS) transistor Q6 to be on/off, and the second-channel second PWM signal received by the second PWM signal receiving terminal P controls a second drive MOS transistor Q2 to be off/on.

On the basis of the above technical solutions, preferably, the transformer unit is a push-pull transformer, and the adjustable voltage output terminal of the DCDC power adjustment unit is connected to a non-dotted terminal of a first primary coil and a dotted terminal of a second primary coil of the push-pull transformer;
the first drive unit includes resistors R10 and R14 and the first drive MOS transistor Q6, where the resistors R10 and R14 are connected in series between the ground and the second PWM signal receiving terminal N, a gate of the first drive MOS transistor Q6 is connected to a node between the resistors R10 and R14, a source of the first drive MOS transistor Q6 is grounded, and a drain of the first drive MOS transistor Q6 is the output terminal of the first drive unit and is connected to a dotted terminal of the first primary coil of the push-pull transformer; and
the second drive unit includes resistors R5 and R13 and the second drive MOS transistor Q2, where the resistors R5 and R13 are connected in series between the ground and the second PWM signal receiving terminal P, a gate of the second drive MOS transistor Q2 is connected to a node between the resistors R5 and R13, a source of the second drive MOS transistor Q2 is grounded, and a drain of the second drive MOS transistor Q2 is the output terminal of the second drive unit and is connected to a non-dotted terminal of the second primary coil of the push-pull transformer.

On the basis of the above technical solutions, preferably, the resonance unit is a series LC resonator, and the series LC resonator includes an inductor T1 and a capacitor C1 that are connected in series in the output circuit of the transformer unit.

On the basis of the above technical solutions, preferably, the sampling unit includes a voltage sampling unit for acquiring a voltage of the output circuit of the transformer unit and a current sampling unit for acquiring a current of the output circuit of the transformer unit, and correspondingly, the output circuit of the transformer unit is provided with a plurality of sampling resistors connected in series; a voltage signal is acquired by a voltage sampling terminal connected to a node between the plurality of sampling resistors, is sent to the voltage sampling unit for filtering and amplification, and then is sent to the main control unit for resonance adjustment; a current signal is acquired by a current sampling terminal connected in series to the output circuit of the transformer unit, is sent to the current sampling unit for filtering and amplification, and then is sent to the main control unit for resonance adjustment.

On the basis of the above technical solutions, preferably, the main control unit includes a main control chip U2 and a main control chip peripheral circuit; the main control chip U2 outputs the first PWM signal to control the DCDC power adjustment unit to output the adjustable voltage, and outputs the two second PWM signals with the complementary duty cycles to the drive unit based on the feedback from the sampling unit that acquires the voltage and the current of the output circuit of the transformer unit.

On the basis of the above technical solutions, preferably, the main control unit further includes an auxiliary chip U1 and an auxiliary chip peripheral circuit; the main control chip U2 of the main control unit sends an instruction to the auxiliary chip U1 based on the feedback from the sampling unit that acquires the voltage and the current of the output circuit of the transformer unit; the auxiliary chip U1 receives the instruction from the main control chip U2 and outputs the two second PWM signals with the complementary duty cycles to the drive unit, where the two second PWM signals with the complementary duty cycles are further fed back to the main control chip U2.

On the basis of the above technical solutions, preferably, the two second PWM signals with the complementary duty cycles are further amplified by a first signal amplifying output circuit and a second signal amplifying output circuit respectively and then are sent to the drive unit.

On the basis of the above technical solutions, preferably, the animal and plant tissue ablation instrument further includes a display unit, a button, a charging interface, a memory, a USB interface unit and/or a touch unit connected to the main control chip U2; when the main control chip U2 is connected to the button and the display unit, the button and a display screen of the display unit are arranged on a surface of the second section of the housing, which is for ease of view.

The present invention has the following beneficial effects.
1. The present invention resolves the followings problems: Existing equipment for rapidly extracting RNA, DNA or protein from biological tissues is expensive, uses a complicated extraction process, requires high operating conditions, wastes resources and time, and has large workload. The ablation instrument of the present invention uses the first PWM signal to control the DCDC power adjustment unit to output the adjustable voltage, so that the output power is adjustable between 0.5 W and 1000 W, and outputs two second PWM signals with complementary duty cycles to the drive unit based on the feedback from the sampling unit that acquires the voltage and current of the output circuit of the transformer unit, so that the ultrasonic resonance point is adjustable between 20 kHz and 200 kHz. The resonance point is adjusted to the resonance point of the ultrasonic vibrator, so that the ultrasonic vibrator operates in a resonance state, with the largest output energy and the largest amplitude. The low-hertz ultrasonic method can quickly and simply ablate the tissue masses into the tissue homogenate, which is convenient for subsequent extraction of RNA, DNA or protein from the tissues, and the cost is low.
2. The present invention resolves the problems of inconvenient structure installation of the existing ultrasonic vibrator and inconvenient hand-holding of the finished structure after installation. The handheld housing of the present invention includes the first section and the second section that are disposed at an obtuse angle to each other. The button and the display screen are arranged on the surface of the second section of the housing, and the end of the first section away from the second section is the front end of the housing. The ultrasonic vibrator is convenient to install, and the finished structure after installation is convenient to hold.
3. The ultrasonic vibrator of the present invention is installed at the front end of the housing through snap-fitting, and is fixed by the first clamping block, the second clamping block, the first fixed cover and the second fixed cover, so that the ultrasonic vibrator is stable in installation and convenient for maintenance and disassembly, and has a good effect and long service life.

### BRIEF DESCRIPTION OF THE DRAWINGS

FIG. 1 is a structural view of an embodiment of the present invention;
FIG. 2 is an exploded structure view of an embodiment of the present invention;
FIG. 3 is a sectional structural view of an embodiment of the present invention;
FIG. 4 is a partial enlarged view of FIG. 3;
FIG. 5 is a system block diagram of an embodiment of the present invention;
FIG. 6 is a circuit principle diagram of a power supply, a DCDC conversion unit and a DCDC power adjustment unit of a control board of an embodiment of the present invention;
FIG. 7 is a circuit principle diagram of a main control unit of a control board of an embodiment of the present invention;
FIG. 8 is a circuit principle diagram of a sampling unit, a display unit and an auxiliary chip of a control board of an embodiment of the present invention;
FIG. 9 is a circuit principle diagram of an interface of a control board of an embodiment of the present invention;
FIG. 10 is a circuit principle diagram of a drive unit, a transformer unit, and a resonance unit of a drive board of an embodiment of the present invention;
FIG. 11 is a diagram of cell suspension according to the present invention; and
FIG. 12 is a diagram of tissue homogenate obtained through processing by an existing tissue homogenizer.

In the figures: 1-housing; 101-upper housing; 102-lower housing; 103-first clamping slot; 2-ultrasonic transducer; 3-amplitude transformer; 4-control board; 5-drive board; 6-button; 7-display screen; 801-first step; 802-first clamping block; 803-second clamping block; 804-first fixed cover; 805-second fixed cover; 806-second step; 807-second clamping slot; 9-hook.

### DETAILED DESCRIPTION OF THE EMBODIMENTS

The present invention will be further described below with reference to the accompanying drawings and specific examples.

### Embodiment 1

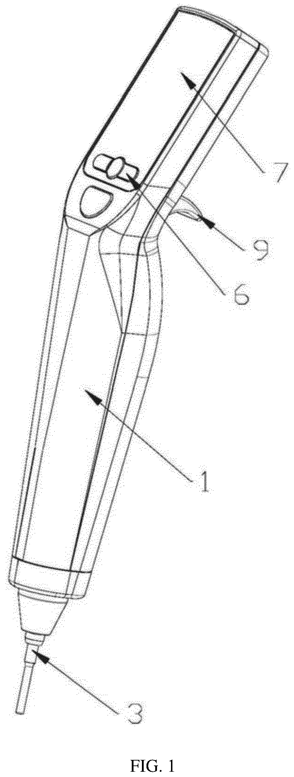

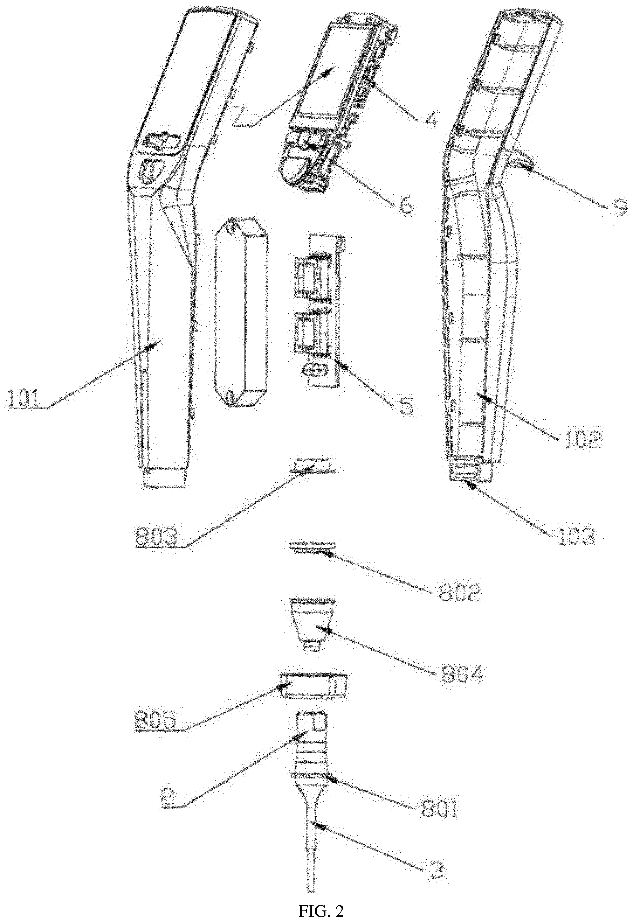

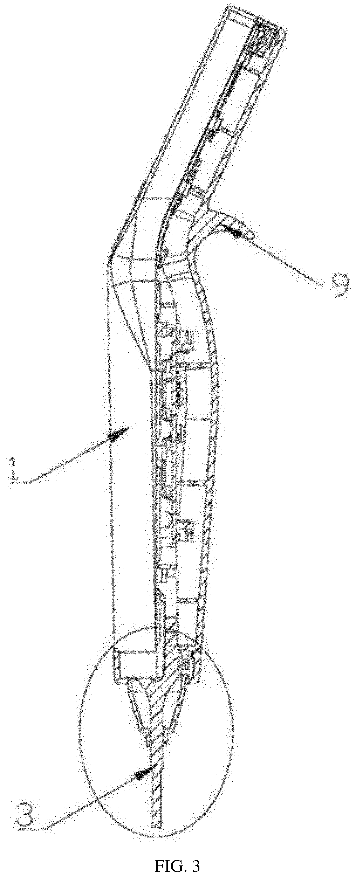

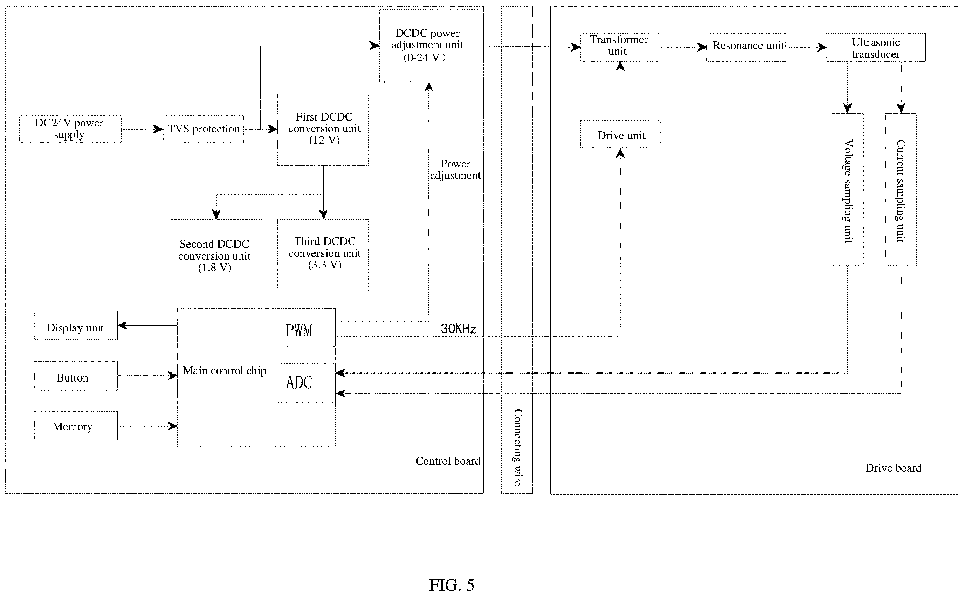

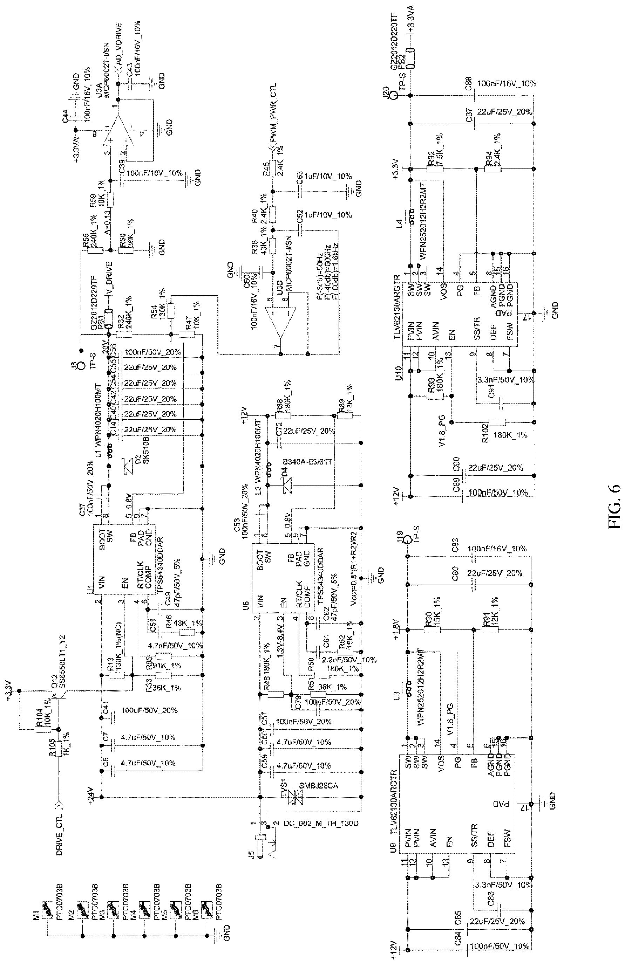

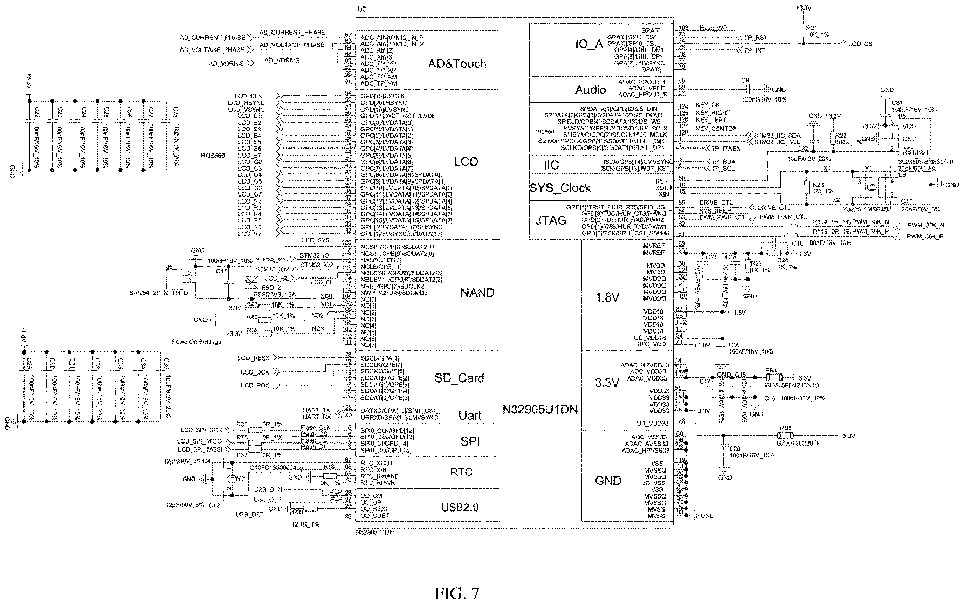

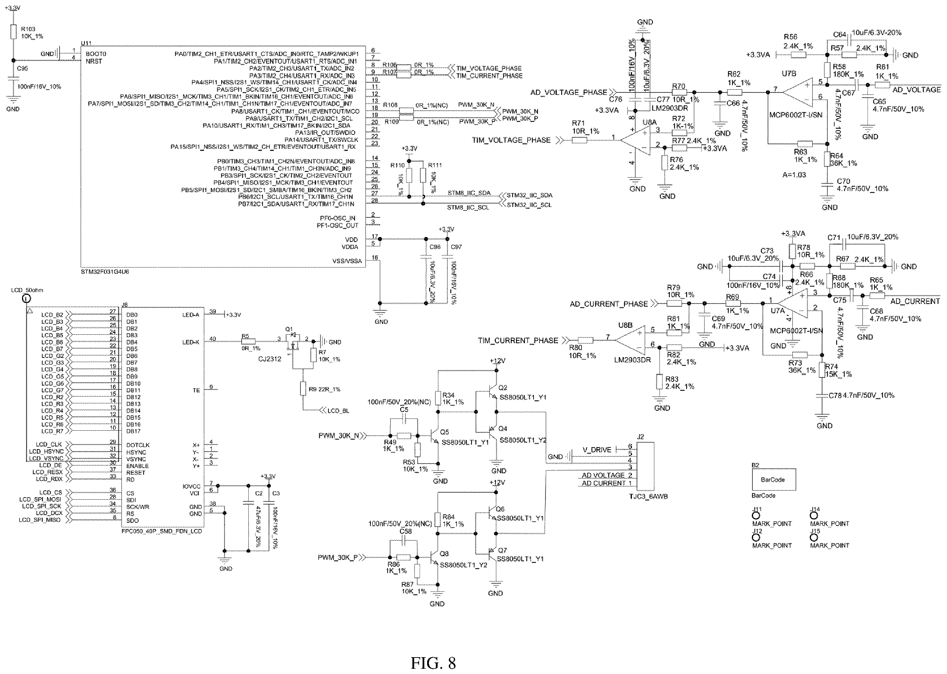

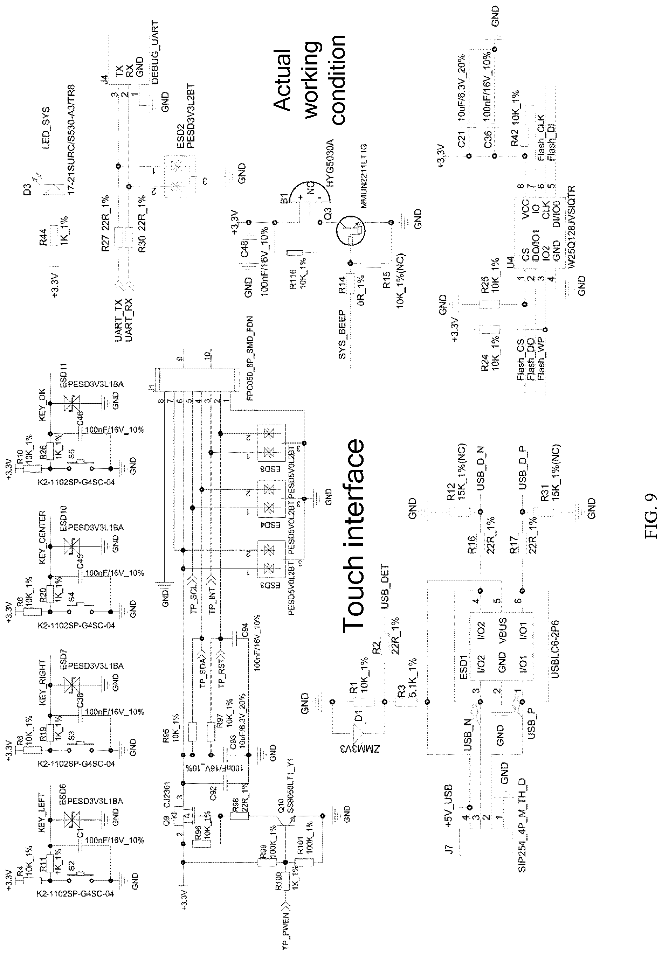

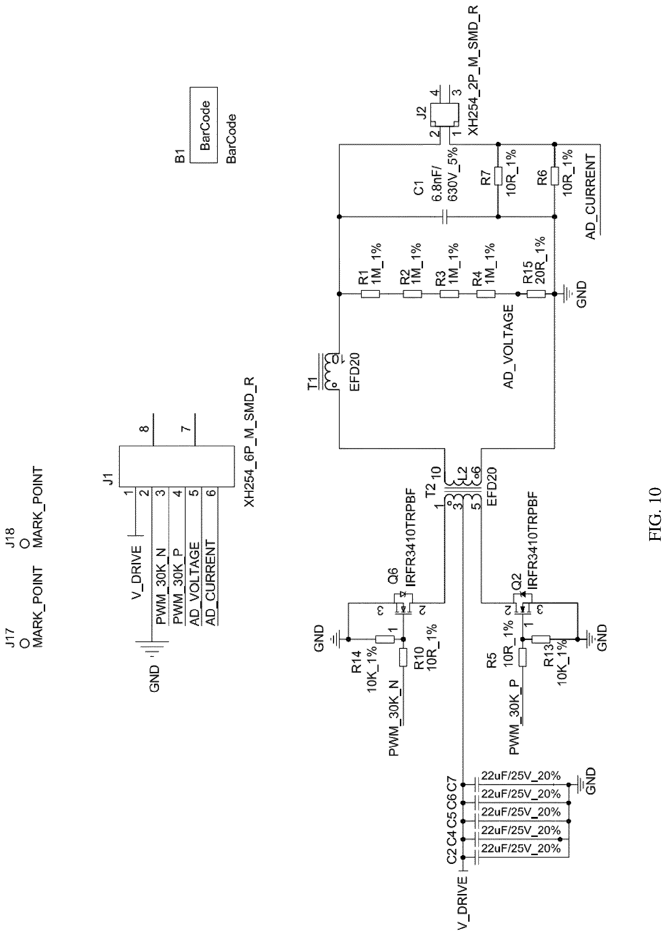

As shown in FIG. 1 to FIG. 10, an ultrasound-based handheld animal and plant tissue ablation instrument includes a housing 1 internally provided with a control board 4 and a drive board 5, and an ultrasonic vibrator fixedly connected to a front end of the housing; the ultrasonic vibrator includes an ultrasonic transducer 2 and an amplitude transformer 3. The amplitude transformer 3 is fixedly connected to a front end of the ultrasonic transducer 2, changes an amplitude of vibration input by the ultrasonic transducer and transmits out the vibration; the control board 4 is electrically connected to the drive board 5, the drive board 5 is electrically connected to the ultrasonic transducer 2, and the control board 4 is connected to a button 6, a display screen 7 and a charging interface; the housing 1 is a handheld housing, and the housing 1 includes a first section and a second section that are disposed at an obtuse angle to each other, the button 6 and the display screen 7 are arranged on a surface of the second section of the housing, and an end of the first section away from the second section is the front end of the housing.

A corresponding button through hole and display screen through hole are arranged on the surface of the second section of the housing, the control board 4 is disposed in the second section of the housing, and the drive board 5 is disposed in the first section of the housing.

The front end of the housing is provided with a connecting hole for fixedly connecting to the ultrasonic vibrator, an inner wall of the connecting hole is provided with one or more first clamping slots 103, and correspondingly, the ultrasonic vibrator is provided with a first step 801 matched with the first clamping slot 103.

In this embodiment, the first step 801 is arranged on the amplitude transformer 3, there are three first clamping slots 103, the first step 801 is clamped into the first clamping slot 103 located in the middle, both sides of the first step 801 are provided with a first clamping block 802 and a second clamping block 803 respectively, and the first step 801 is clamped in the first clamping slot 103 through the first clamping block 802 and the second clamping block 803. The first clamping slot 103 is an annular clamping slot, the first step 801 is an annular step, the first clamping block 802 and the second clamping block 803 are both annular clamping blocks, the first clamping block 802 and the second clamping block 803 both sleeve over an outer wall of the ultrasonic vibrator, and the second clamping block 803 also extends through the first clamping slot 103 at the rear end.

In this embodiment, a front end of the amplitude transformer 3 provided with the first step is also sleeved with a first fixed cover 804, and a rear end of the first fixed cover 804 is clamped in the first clamping slot 103 at the front end.

In this embodiment, the housing includes an upper housing 101 and a lower housing 102 that are engaged with each other. A front end of the upper housing 101 and the lower housing 102 are also sleeved with a second fixed cover 805, an inner wall of the second fixed cover 805 is provided with two second steps 806, and an outer wall of the front end of the upper housing 101 and the lower housing 102 is also provided with a second clamping slot 807 matched with the second step 806 at the rear end. The second step 806 at the front end wraps the front end of the housing, and further extends to the first clamping slot 103 at the front end to clamp a rear end of the first fixed cover 804.

In this embodiment, the control board 4 and the drive board 5 are fixed in the housing 1 by screws. The drive board 5 is further provided with a protective housing, and a surface of the display screen 7 is also pasted with a tempered glass film.

In this embodiment, a hook 9 is arranged on the outer wall of the housing.

It should be noted that the amplitude transformer and ultrasonic transducer are existing products, and their internal structures are relatively mature technologies. Details are not described herein. The above-mentioned front and rear ends are relative positions, not absolute positions.

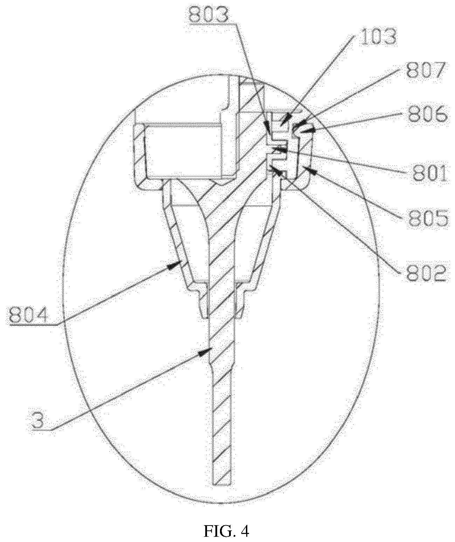

As shown in FIG. 4 to FIG. 9, the control board is provided with a power supply, a DCDC conversion unit, a main control unit, a DCDC power adjustment unit and a sampling unit, and the drive board is provided with a drive unit, a transformer unit and a resonance unit.

A voltage of the power supply is converted through the DCDC conversion unit and the DCDC power adjustment unit and then is supplied to the animal and plant tissue ablation instrument; the main control unit outputs a first PWM signal to control the DCDC power adjustment unit to output an adjustable voltage, and outputs two second PWM signals with complementary duty cycles to the drive unit based on feedback from the sampling unit that acquires the voltage and the current of an output circuit of the transformer unit, the transformer unit transforms the voltage, and then the resonance unit arranged in the output circuit of the transformer unit adjusts a resonance point to a resonance point of the ultrasonic vibrator, so that the ultrasonic vibrator arranged in the output circuit of the transformer unit operates in a resonance state.

The animal and plant tissue ablation instrument will be further elaborated.

The power supply is a DC24V power supply, and the DCDC conversion unit includes a first DCDC conversion unit, a second DCDC conversion unit, and a third DCDC conversion unit.

The first DCDC conversion unit includes transient voltage suppressor (TVS) protection, a first voltage input filter, a first voltage conversion chip and a first voltage output filter. A voltage of the DC24V power supply sequentially passes the TVS protection, the first voltage input filter, the first voltage conversion chip and the first voltage output filter and is converted into an output voltage of 12 V, which is supplied to the main control unit and the drive unit.

A circuit principle of the first DCDC conversion unit is described in detail: The voltage of the DC24V power supply passes a protective diode TVS1 for TVS protection, passes three capacitors C59, C60 and C57 connected in parallel with the DC24V power supply for voltage input filtering, passes voltage divider resistors R48 and R51 connected in series with the DC24V power supply for voltage sampling, and then is input into an enable pin EN of a first voltage conversion chip U6. The first voltage conversion chip U6 is TPS54340, and an upper tube drive signal reference point pin SW of the first voltage conversion chip U6 is connected to a 12V voltage output terminal through an energy storage inductor L2. Voltage divider resistors R88 and R89 connected in series with the 12V voltage output terminal sample a voltage and input the voltage to a reference voltage pin FB of the first voltage conversion chip U6. A capacitor C72 connected to the 12V voltage output terminal performs voltage output filtering. The upper tube drive signal reference point pin SW of the first voltage conversion chip U6 is further connected to a freewheeling diode D4, the enable pin EN is further connected to a soft start capacitor C79, a resistor timing/external clock pin RT/CLK is connected to a frequency divider resistor R50, and a frequency compensation pin COMP is connected to capacitors C61 and C62 and a resistor R52 that are used to adjust a circuit and stabilize voltage output. The capacitor C62 is connected in parallel with the capacitor C61 and the resistor R52 that are connected in series.

The second DCDC conversion unit includes a second voltage input filter, a second voltage conversion chip and a second voltage output filter. The 12V input voltage sequentially passes the second voltage input filter, the second voltage conversion chip and the second voltage output filter, and is converted into a 1.8V output voltage, which is supplied to the main control unit.

The circuit principle of the second DCDC conversion unit is described in detail: The 12V input voltage sequentially passes two capacitors C84 and C85 connected in parallel with a 12V voltage input terminal for voltage input filtering. A second voltage conversion chip U9 is TLV62130ARGTR, and an MOS tube drive signal reference point pin SW of the second voltage conversion chip U9 is connected to a 1.8V voltage output terminal through an energy storage inductor L3. Voltage divider resistors R90 and R91 connected in series to the 1.8V voltage output terminal perform voltage sampling and input the voltage into a reference voltage pin FB of the second voltage conversion chip U9. Capacitors C80 and C83 connected in parallel with the 1.8V voltage output terminal perform voltage output filtering. An internal power supply pin PVIN of the second voltage conversion chip U9, an internal control circuit power supply pin AVIN, and an enable pin EN are connected to the 12V voltage input terminal, a soft-start/tracking pin SS/TR is connected to a soft-start capacitor C86, an adjustment output pin DEF and a frequency selection pin FSW are both grounded, and an output voltage acquisition pin VOS is connected to the 1.8V voltage output terminal.

The third DCDC conversion unit includes a third voltage input filter, a third voltage conversion chip, and a third voltage output filter. The 12V input voltage sequentially passes the third voltage input filter, the third voltage conversion chip, and the third voltage output filter, and is converted into a 3.3V output voltage, which is supplied to the main control unit and the sampling unit.

The circuit principle of the third DCDC conversion unit is described in detail: The 12V input voltage passes two capacitors C89 and C90 connected in parallel with the 12V voltage input terminal for voltage input filtering. The third voltage conversion chip U10 is TLV62130ARGTR, and an MOS tube drive signal reference point pin SW of the third voltage conversion chip U10 is connected to a 3.3V voltage output terminal through an energy storage inductor L4. Voltage divider resistors R92 and R94 connected in series to the 3.3V voltage output terminal sample a voltage sampling and input the voltage to a reference voltage pin FB of the third voltage conversion chip U10. Capacitors C87 and C88 connected in parallel with a 3.3V voltage output terminal perform voltage output filtering. An internal power supply pin PVIN and an internal control circuit power supply pin AVIN of the third voltage conversion chip U10 are further connected to the 12V voltage input terminal. Voltage divider resistors R93 and R102 connected in series with the 12V voltage input terminal perform voltage sampling and input the voltage to an enable pin EN of the third voltage conversion chip U10, and the enable pin EN of the third voltage conversion chip U10 is further connected to a normal output indication signal pin PG of the second voltage conversion chip U9. When the second DCDC conversion unit does not work normally, the third DCDC conversion unit stops working. A soft-start/tracking pin SS/TR is connected to a soft-start capacitor C91, an adjustment output pin DEF and a frequency selection pin FSW are both grounded, and an output voltage acquisition pin VOS is connected to the 3.3V voltage output terminal.

The DCDC power adjustment unit includes a switch control signal receiving circuit, a first PWM signal receiving circuit, and a fourth DCDC conversion unit. The fourth DCDC conversion unit includes a fourth voltage input filter, a fourth voltage conversion chip and a fourth voltage output filter. The fourth DCDC conversion unit converts, through a switch control signal and a first PWM signal sent by the main control unit, the DC24V power supply into a 0-24V adjustable voltage for output.

The circuit principle of the DCDC power adjustment unit is described in detail:

The circuit principle of the fourth DCDC conversion unit is as follows: The voltage of the DC24V power supply sequentially passes three capacitors C6, C7 and C41 connected in parallel with the DC24V power supply for voltage input filtering. The fourth voltage conversion chip U1 is TPS54340, and an upper tube drive signal reference point pin SW of the fourth voltage conversion chip U1 is connected to the adjustable voltage output terminal through an energy storage inductor L1. Voltage divider resistors R32 and R47 connected in series with the adjustable voltage output terminal sample a voltage and input the voltage to a reference voltage pin FB of the fourth voltage conversion chip U1, capacitors C14, C40, C42, C54, C55 and C56 connected in parallel with the adjustable voltage output terminal perform output filtering on the adjustable voltage. The upper tube drive signal reference point pin SW of the fourth voltage conversion chip U1 is further connected to a freewheeling diode D2, an enable pin EN is connected to a resistor R33, a resistor timing/external clock pin RT/CLK is connected to a frequency divider resistor R85, and a frequency compensation pin COMP is connected to the capacitor C51, the capacitor C49 and the resistor R46 that are used to adjust a circuit and stabilize voltage output. The capacitor C51 and the resistor R46 are connected in series and then are connected with the capacitor C49 in parallel.

The circuit principle of the switch control signal receiving circuit is as follows: A switch control signal receiving terminal is connected to the main control unit to receive the switch control signal, resistors R104 and R105 are connected in series between the 3.3V voltage terminal and the switch control signal receiving terminal. The emitter of a transistor Q12 is connected to the 3.3V voltage terminal, the base of the transistor Q12 is connected to a node between the resistor R104 and the resistor R105, and the collector of the transistor Q12 is connected to the enable pin EN of the fourth voltage conversion chip U1. When the switch control signal is L, the transistor Q12 is turned on, and the fourth voltage conversion chip U1 is activated to work.

The circuit principle of the first PWM signal receiving circuit is as follows: The first PWM signal received by the first PWM signal receiving terminal is filtered through an RC filter and then input to a non-inverting input terminal of a first voltage follower U3B, and an output terminal of the first voltage follower U3B is connected to a node between the voltage divider resistors R32 and R47 through a voltage divider resistor R54. The resistor R45, the capacitor C63, the resistor R40 and the capacitor C52 form the RC filter, and then the voltage is input to the non-inverting input terminal of the first voltage follower U3B through a resistor R36. The non-inverting input terminal of the first voltage follower U3B is further connected to a capacitor C50.

The animal and plant tissue ablation instrument further includes an output voltage sampling unit for acquiring the adjustable voltage of the DCDC power adjustment unit, and the acquired adjustable voltage is sent to the main control unit through a second voltage follower U3A.

The circuit principle of the output voltage sampling unit is described in detail: Voltage divider resistors R55 and R60 connected in series with the adjustable voltage output terminal perform voltage sampling, the RC filter filters the voltage, and then the voltage is input to a non-inverting input terminal of the second voltage follower U3A. A positive voltage terminal of the second voltage follower U3A is connected to the 3.3V voltage terminal, and an output terminal of the second voltage follower U3A is connected to the main control unit. A resistor R59 and a capacitor C39 form the RC filter, and the 3.3V voltage terminal is further connected to a capacitor C44. The output terminal of the second voltage follower U3A is further connected to a capacitor C43.

The drive unit includes a first drive unit and a second drive unit, the first drive unit includes a first drive MOS transistor Q6, and the second drive unit includes a second drive MOS transistor Q2. A second PWM signal receiving terminal N of the first drive unit receives the first-channel second PWM signal sent by the main control unit, an output terminal of the first drive unit is connected to a dotted terminal of a primary coil of the transformer unit, a second PWM signal receiving terminal P of the second drive unit receives the second-channel second PWM signal sent by the main control unit, an output terminal of the second drive unit is connected to a non-dotted terminal of the primary coil of the transformer unit, the first-channel second PWM signal received by the second PWM signal receiving terminal N controls the first drive MOS transistor Q6 to be on/off, and the second-channel second PWM signal received by the second PWM signal receiving terminal P controls the second drive MOS transistor Q2 to be off/on.

The circuit principle of the drive unit and the transformer unit is described in detail: The transformer unit is a push-pull transformer, and the adjustable voltage output terminal of the DCDC power adjustment unit is connected to a non-dotted terminal of a first primary coil and a dotted terminal of a second primary coil. Resistors R10 and R14 are connected in series between the ground and the second PWM signal receiving terminal N. A gate of the first drive MOS transistor Q6 is connected to a node between the resistors R10 and R14, a source is grounded, and a drain is the output terminal of the first drive unit and is connected to a dotted terminal of the first primary coil of the push-pull transformer. Resistors R5 and R13 are connected in series between the ground and the second PWM signal receiving terminal P. A gate of the second drive MOS transistor Q2 is connected to a node between the resistors R5 and R13, a source is grounded, and a drain is the output terminal of the second drive unit and is connected to a non-dotted terminal of the second primary coil of the push-pull transformer.

When the first-channel second PWM signal received by the second PWM signal receiving terminal N is H and the second-channel second PWM signal received by the second PWM signal receiving terminal P is L, the first drive MOS transistor Q6 is controlled to be turned on, the second drive MOS transistor Q2 is turned off, an input circuit of the first primary coil is connected and an input current direction is from the non-dotted terminal to the dotted terminal of the first primary coil, an input circuit of the second primary coil is cut off, and an output current direction of an output circuit of a secondary coil is from a dotted terminal to a non-dotted terminal of the secondary coil.

When the first-channel second PWM signal received by the second PWM signal receiving terminal N is L and the second-channel second PWM signal received by the second PWM signal receiving terminal P is H, the first drive MOS transistor Q6 is controlled to be turned off, the second drive MOS transistor Q2 is turned on, the input circuit of the second primary coil is connected and an input current direction is from the dotted terminal to the non-dotted terminal of the second primary coil, an input circuit of the first primary coil is cut off, and an output current direction of an output circuit of the secondary coil is from the non-dotted terminal to the dotted terminal of the secondary coil. The input DC voltage is converted into an AC waveform with a frequency of 30 kHz and a voltage of hundreds of volts, which facilitates subsequent resonance unit.

The resonance unit is disposed in the output circuit of the push-pull transformer. The resonance unit is a series LC resonator, and the series LC resonator includes an inductor T1 and a capacitor C1 that are connected in series in the output circuit of the transformer unit. The inductor T1 uses EFD20.

The ultrasonic transducer is disposed in the output circuit of the push-pull transformer. In this embodiment, the ultrasonic transducer is connected through an interface J2.

The sampling unit acquires a voltage and current of the output circuit of the push-pull transformer. The sampling unit includes a voltage sampling unit and a current sampling unit.

The voltage sampling unit acquires the voltage of the output circuit of the push-pull transformer. The output circuit of the push-pull transformer is provided with sampling resistors R1, R2, R3, R4 and R15 connected in series. A voltage sampling terminal is connected to a node between the resistors R4 and R15, and an acquired voltage signal is sent to the voltage sampling unit for filtering and amplification, and then sent to the main control unit for resonance adjustment.

The circuit principle of the voltage sampling unit is described in detail: The acquired voltage signal is filtered through the RC filter, a capacitor C67 performs DC blocking on the voltage signal, and then the voltage signal is input to a non-inverting input terminal of a first operational amplifier U7B. A negative feedback resistor R63 is connected between an inverting input terminal and output terminal of the first operational amplifier U7B. The signal is filtered through an RC filter at the output terminal of the first operational amplifier U7B, and then input to a non-inverting input terminal of a first comparator U8A through a resistor R72. Voltage divider resistors R77 and R76 connected in series between the 3.3V voltage terminal and the ground perform voltage sampling and input the voltage to an inverting input terminal of the first comparator U8A. An output terminal of the first comparator U8A is connected to the main control unit through a resistor R71. The resistor R72 is connected to the non-inverting input terminal of the first comparator U8A, and the other end of the resistor R72 is connected to the main control unit through a resistor R70. A resistor R61 and a capacitor C65 form an RC filter to filter the acquired voltage signal, and a resistor R62 and a capacitor C66 form an RC filter to filter a signal output from the output terminal of the first operational amplifier U7B. The inverting input terminal of the first operational amplifier U7B is further connected in series with a resistor R64 and a capacitor C70, and the non-inverting input terminal of the first operational amplifier U7B is further connected to a DC 3.3V voltage terminal. The DC 3.3V voltage is divided through voltage divider resistors R56, R58 and R57, to generate a divided voltage, and the divided voltage is input to the non-inverting input terminal of the first operational amplifier U7B. The divided voltage is further filtered through a capacitor C64. One end of the resistor R58 is connected to the non-inverting input terminal of the first operational amplifier U7B and the other end is connected to the resistor R56. The resistor R56 is connected to the 3.3V voltage terminal. One end of the resistor R57 is connected to a node between the resistors R56 and R58 and the other end is grounded. A positive voltage terminal of the first comparator U8A is connected to the 3.3V voltage terminal, and the 3.3V voltage is filtered through parallel capacitors C76 and C77.

The current sampling unit acquires a current of the output circuit of the push-pull transformer. A current sampling terminal is connected in series with the output circuit of the push-pull transformer, and then sends the acquired current signal to the current sampling unit for filtering and amplification. Then the signal is sent to the main control unit for resonance adjustment.

The circuit principle of the current sampling unit is described in detail: The acquired current signal is filtered through an RC filter, a capacitor C75 performs DC blocking on the current signal, and then the current signal is input to a non-inverting input terminal of a second operational amplifier U7A. A negative feedback resistor R73 is connected between an inverting input terminal and output terminal of the second operational amplifier U7A. The signal is filtered through an RC filter at the output terminal of the second operational amplifier U7A, and then input to a non-inverting input terminal of a second comparator U8B through a resistor R81. Voltage divider resistors R82 and R83 connected in series between the 3.3V voltage terminal and the ground sample a voltage and input the voltage to an inverting input terminal of the second comparator U8B. An output terminal of the second comparator U8B is connected to the main control unit through a resistor R80. A resistor R81 is connected to the non-inverting input terminal of the second comparator U8B, and the other end is connected to the main control unit through a resistor R79. A resistor R65 and a capacitor C68 form an RC filter to filter the acquired current signal, and a resistor R69 and a capacitor C69 form an RC filter to filter a signal output by the output terminal of the second operational amplifier U7A. The inverting input terminal of the second operational amplifier U7A is further connected in series with a resistor R74 and a capacitor C78, and the non-inverting input terminal of the second operational amplifier U7A is further connected to the DC 3.3V voltage terminal. The DC 3.3V voltage is divided through voltage divider resistors R78, R66, R67 and R68, to generate a divided voltage, and the divided voltage is input to the non-inverting input terminal of the second operational amplifier U7A. The divided voltage is filtered through a capacitor 71. One end of the resistor R68 is connected to the non-inverting input terminal of the second operational amplifier U7A and the other end is connected to the resistors R78 and R66 that connected in series with the 3.3V voltage terminal. One end of the resistor R67 is connected to a node between the resistors R66 and R68 and the other end is grounded, a positive voltage terminal of the second comparator U8B is connected to the 3.3V voltage terminal through the resistor R78, and the 3.3V voltage is filtered through parallel capacitors C73 and C74.

The main control unit outputs the first PWM signal to control the DCDC power adjustment unit to output the adjustable voltage, and outputs two second PWM signals with complementary duty cycles to the first drive MOS transistor Q6 and the second drive MOS transistor Q2 of the drive unit based on the feedback from the sampling unit that acquires the voltage and current of the output circuit of the transformer unit.

The main control unit includes a main control chip U2 and a main control chip peripheral circuit. The main control chip U2 is N32905U1DN with an ARM9 core and a main frequency of 200 MHz. The main control chip peripheral circuit includes a system clock, a reset, etc.

The N32905U1DNN3290x is based on an ARM926EJ-S CPU core and integrates a JPEG codec, a CMOS sensor interface, a 32-channel sound processing unit (SPU), an analog-to-digital converter (ADC), a digital-to-analog converter (DAC), which can meet various application requirements while saving BOM costs. ARM926@200MHz synchronizes with a dynamic random access memory (DRAM), a 2D BitBLT accelerator, a CMOS image sensor interface, and a liquid crystal display (LCD) panel interface. The maximum resolution of N32905U1DNN3290x is XVGA(1,024x768)@TFT LCD panel. The 2D BitBLT accelerator accelerates graphics calculations, smooths rendering, and offloads the CPU to reduce power consumption.

In order to meet the different requirements on the overall BOM costs of the system, DRAMs of different sizes and the N3290x main SoC are stacked into one package, that is, a multi-chip package (MCP) is used. N32905U1DNN3290x is specially designed by 1Mbitx16 3.3V SDRAM. N32905U1DNN3290x is specially designed by 4Mbitx16 1.8V DDR SDRAM. A 16Mbitx16 1.8V DDR2SDRAM is stacked inside N32905U1DNN3290x to ensure higher performance and minimize system design work such as electromagnetic interference (EMI) and noise coupling. A double-layer PCB is adopted and damping resistors and EMI protection assemblies are eliminated, so that the total BOM costs can be reduced.

The above technical solutions have been fully disclosed and can be implemented. The present invention resolves the followings problems: Existing equipment for rapidly extracting RNA, DNA or protein from biological tissues is expensive, uses a complicated extraction process, requires high operating conditions, wastes resources and time, and has large workload. The ablation instrument of the present invention uses the first PWM signal to control the DCDC power adjustment unit to output the adjustable voltage, so that the output power is adjustable between 0.5 W and 1000 W, and outputs two second PWM signals with complementary duty cycles to the drive unit based on the feedback from the sampling unit that acquires the voltage and current of the output circuit of the transformer unit, so that the resonance ultrasonic frequency is adjustable between 20 kHz and 200 kHz. The resonance point is adjusted to the resonance point of the ultrasonic vibrator, so that the ultrasonic vibrator operates in a resonance state, with the largest output energy and the largest amplitude. The low-hertz ultrasonic method can quickly and simply ablate the tissue block into the tissue homogenate, which is convenient for subsequent extraction of RNA, DNA or protein from the tissues.

In specific applications, the main control unit of the present invention also includes an auxiliary chip U1 and an auxiliary chip peripheral circuit, and the auxiliary chip U1 is STM32F031G4U6.

Features of STM32F031G4U6 are as follows: Core: 32-bit CPU with frequency up to 48 MHz; memory: 16 to 32 KB of flash memory, and 4 KB of SRAM with hardware parity; cyclic redundancy check (CRC) calculation unit; reset and power management: digital and I/O power supply: 2.0-3.6V, analog power supply: V_{DDA}=V_{DD} to 3.6 V, power-on/power-down reset (POR/PDR), programmable voltage detector (PVD), low power modes including sleep, stop and standby, and VBAT power supply for RTC and backup registers; clock management: 4-32 MHz crystal oscillator, 32 kHz oscillator for RTC with calibration, internal 8 MHz RC with x6PLL option, internal 40 kHz RC oscillator; up to 39 fast I/Os: all mappable on external interrupt vectors, up to 26 I/Os with 5V tolerant capability; five-channel DMA controller: one 12-bit, 1.0 µs ADC (up to 10 channels), conversion range: 0-3.6 V, separate analog supply 2.4-3.6 V; up to 9 timers: one 16-bit 7- channel advanced control timer, for 6-channel PWM output, dead time, power generation and emergency stop, one 32-bit and one 16-bit timers, with up to four IC/OC, usable for IR control decoding, one 16-bit timer, with two IC/OC, one OCN, dead time and emergency stop, one 16-bit timer, with IC/OC and OCN, dead time, emergency stop and modulator gate for IR control, one 16-bit timer with one IC/OC, independent and system watchdog timer, SysTick timer: 24-bit downcounter; calendar RTC with alarm and periodic wake-up from stop/standby; communication interface: one I²C interface supporting fast mode plus (IMbit/s) with 20mA current sink, SMBus/PMBus, wake-up from stop, one USART supporting master synchronous SPI and modem control, ISO7816 interface, LIN, IrDA, auto baud rate detection and wakeup function, one SPI (18 Mbit/s) with 4 to 16 programmable bit frame, and with I²S interface multiplexed; serial wire debug (SWD); 96-bit unique ID; extended temperature range: -40°C to +105°C; all packages ECOPACK^{®} 2.

The main control chip U2 of the main control unit sends an instruction to the auxiliary chip U1 based on the feedback from the sampling unit that acquires the voltage and current of the output circuit of the transformer unit, and the auxiliary chip U1 receives the instruction from the main control chip U2 and outputs two second PWM signals with frequency of 30 kHz and complementary duty cycles to the first drive MOS transistor Q6 and the second drive MOS transistor Q2 of the drive unit. The two second PWM signals with frequency of 30 kHz and complementary duty cycles are further fed back to the main control chip U2. In addition, signals output from the output terminal of the first comparator U8A and the output terminal of the second comparator U8B are sent to the auxiliary chip U1.

The two second PWM signals with frequency of 30 kHz and complementary duty cycles are further amplified by a first signal amplifying output circuit and a second signal amplifying output circuit respectively and then sent to the first drive MOS transistor Q6 and the second drive MOS transistor Q2 of the drive unit.

The circuit principle of the first signal amplifying output circuit is described in detail: The second PWM signal is input to a base of a first amplifying transistor Q5 for amplification through voltage divider resistors R49 and R53 that are connected in series, a collector of the first amplifying transistor Q5 is connected to a 12V voltage terminal through a resistor R34, and an emitter of the first amplifying transistor Q5 is grounded. The collector of the first amplifying transistor Q5 is connected to bases of a first output transistor Q2 and a second output transistor Q4, the first output transistor Q2 is a P-type transistor, and the second output transistor Q4 is an N-type transistor. A collector of the first output transistor Q2 is connected to a 12V voltage terminal, and the amplified first-channel second PWM signal is output to the second PWM signal receiving terminal N of the first drive MOS transistor Q6 from a connecting node between an emitter of the first output transistor Q2 and an emitter of the second output transistor Q4.

When the second PWM signal is H, the first output transistor Q2 is turned on, and the second output transistor Q4 is turned off; when the second PWM signal is L, the first output transistor Q2 is turned off, and the second output transistor Q4 is turned on. Therefore, the amplified square wave first-channel second PWM signal is output.

The circuit principle of the second signal amplifying output circuit is described in detail: The second PWM signal is input to a base of a second amplifying transistor Q8 for amplification through voltage divider resistors R86 and R87 that are connected in series, a collector of the second amplifying transistor Q8 is connected to the 12V voltage terminal through a resistor R84, an emitter of the second amplifying transistor Q8 is grounded, and a collector of the second amplifying transistor Q8 is connected to bases of a third output transistor Q6 and a fourth output transistor Q7. The third output transistor Q6 is a P-type transistor, and the fourth output transistor Q7 is an N-type transistor. A collector of the third output transistor Q6 is connected to the 12V voltage terminal, and the amplified second-channel second PWM signal is output to the second PWM signal receiving terminal P of the second drive MOS transistor Q2 from a connecting node between an emitter of the third output transistor Q6 and an emitter of the fourth output transistor Q7.

When the second PWM signal is H, the third output transistor Q6 is turned on, and the fourth output transistor Q7 is turned off; when the second PWM signal is L, the third output transistor Q6 is turned off, and the fourth output transistor Q7 is turned on. Therefore, the amplified square wave second-channel second PWM signal is output.

A display screen 7 is an LCD display screen. The LCD display screen is connected to a display chip J8 to form a display unit. The display chip J8 is connected to the main control chip U2. The display chip J8 is FPC050. The display chip J8 is connected to the 3.3V voltage terminal. A signal LCD_BL from the main control chip U2 is amplified by a MOS transistor Q1 and sent to the display chip J8.

A button 6 is connected to the main control chip U2. In this embodiment, there are four buttons, namely a first button, a second button, a third button, and a fourth button. The first button is a left button, the second button is a right button, the third button is a middle button, and the fourth button is an OK button. The first button includes a pull-up resistor R4 and a pull-down resistor R11, the pull-up resistor R4 is connected to the 3.3V voltage terminal, a node between the pull-up resistor R4 and the pull-down resistor R11 is connected to one end of a button S2, and the other end of the button S2 is grounded. The other end of the pull-down resistor R11 is connected to the main control chip U2. When the button S2 is pressed, the pull-down resistor R11 is only 1 K, and the first button outputs a low level. When the button S2 is released, the output is pulled up by the pull-up resistor R4 and the pull-down resistor R11. The first button outputs a high level. The second button includes a pull-up resistor R6 and a pull-down resistor R19. The pull-up resistor R6 is connected to the 3.3V voltage terminal. A node between the pull-up resistor R6 and the pull-down resistor R19 is connected to one end of a button S3, and the other end of the button S3 is grounded. The other end of the pull-down resistor R19 is connected to the main control chip U2. When the button S3 is pressed, the second button outputs a low level. When the button S3 is released, the output is pulled up by the pull-up resistor R6 and the pull-down resistor R19. The second button outputs a high level. The third button includes a pull-up resistor R8 and a pull-down resistor R20, the pull-up resistor R8 is connected to the 3.3V voltage terminal, a node between the pull-up resistor R8 and the pull-down resistor R20 is connected to one end of the button S4, and the other end of the button S4 is grounded. The other end of the pull-down resistor R20 is connected to the main control chip U2. When the button S4 is pressed, the third button outputs a low level. When the button S4 is released, the output is pulled up by the pull-up resistor R8 and the pull-down resistor R20. The third button outputs a high level. The fourth button includes a pull-up resistor R10 and a pull-down resistor R26, the pull-up resistor R10 is connected to the 3.3V voltage terminal, a node between the pull-up resistor R10 and the pull-down resistor R26 is connected to one end of a button S5, and the other end of the button S5 is grounded. The other end of the pull-down resistor R26 is connected to the main control chip U2. When the button S5 is pressed, the fourth button outputs a low level. When the button S5 is released, the output is pulled up by the pull-up resistor R10 and the pull-down resistor R26. The fourth button outputs a high level.

The main control chip U2 is further connected to a memory, and the memory uses an SPI-FLASH device to store parameters including resonance parameters, setting parameters, and so on.

The main control chip U2 is further connected to a USB interface unit. The USB interface unit includes a USB chip ESDI connected to the main control chip U2 and an interface J7 connected to the USB chip ESD1. The USB chip ESDI is USBLC6, and the interface J7 is SIP254.

The main control chip U2 is further connected to a touch unit. The touch unit includes voltage divider resistors R99 and R101 connected in series between the 3.3V voltage terminal and the ground, a resistor R100 connected to a node between the resistors R99 and R101, and a transistor Q10 whose base is connected to the node between the resistors R99 and R101. Voltage divider resistors R98 and R96 are connected in series between a collector of the transistor Q10 and the 3.3V voltage terminal. A node between the voltage divider resistors R98 and R96 is connected to a gate of an MOS transistor Q9, a source of the MOS transistor Q9 is connected to the 3.3V voltage terminal, and a drain of the MOS transistor Q9 is connected to a touch interface J1. The touch interface J1 is connected to the main control chip U2, the touch interface J1 is connected to a touch panel, and a power enable signal TP_PWEN sent by the main control chip U2 arrives at the base of the transistor Q10 through the resistor R100. When the power enable signal TP_PWEN is H, the transistor Q10 is turned on, and the MOS transistor Q9 is turned on.

The main control chip U2 is further connected to an external interface J4. The main control chip U2 is further connected to a buzzer.

### Embodiment 2:

This embodiment provides a method for rapidly extracting RNA, DNA or protein by using the ultrasound-based handheld animal and plant tissue ablation instrument. The method includes the following steps: A: Prepare cell suspension. B: Extract RNA, DNA or protein. The step A includes: after a tissue sample is mixed with solution, perform ultrasonic ablation by using the handheld animal and plant tissue ablation instrument based on the principle of ultrasonic, to obtain the cell suspension.

The low-hertz ultrasonic method can quickly and simply ablate the tissue masses into the tissue suspension, which is convenient for subsequent extraction of RNA, DNA or protein from the tissues. The RNA, DNA or protein, especially the RNA and protein, can be extracted from the tissues with only a small quantity of tissues. The method is simple to operate, saves extraction time, and greatly shortens the time from tissue masses to cell suspension. All operations can be completed by only one person, which greatly reduces manpower and material resources, and improves work efficiency.

In order to achieve the best homogenization effect of the tissues, the conditions for selecting ultrasonic ablation are that the power is 0.5-1000 W and the ultrasonic frequency is 20-200 kHz.

The solution in the step A is an RNA extracting solution, a protein extracting solution, a DNA extracting solution, physiological saline or a buffer solution.

In the ultrasonic ablation process, in order to facilitate subsequent procedures, different solutions may be selected for different subsequent procedures, to reduce operation steps and reducing extraction time. When the RNA, DNA or protein is to be extracted, selecting the RNA extracting solution, the DNA extracting solution, or the protein extracting solution can greatly reduce the extraction time while ensuring the extraction effect. From a general point of view, in the present invention, the physiological saline or the buffer solution can be used when the cell suspension is obtained through ultrasonic ablation.

In an embodiment, when the solution in the step A is the RNA extracting solution, the protein extracting solution, or the DNA extracting solution, the step B may include extracting the RNA, protein, or DNA respectively according to the conventional operation method of the RNA extracting solution, the protein extracting solution or the DNA extracting solution.

When the RNA extracting solution, the protein extracting solution or the DNA extracting solution is used as a solution to obtain the cell suspension through ultrasonic ablation, in the subsequent specific steps of extracting the RNA, DNA or protein, the extraction operations may be performed according to operating instructions of a reagent corresponding to the RNA extracting solution, the DNA extracting solution or the protein extracting solution, or other conventional extraction methods suitable for extraction may be used.

In another embodiment, when the solution in the step A is the physiological saline or the buffer solution, the step B includes mixing the cell suspension with an extraction amount of the RNA extracting solution, the protein extracting solution or the DNA extracting solution, and then extracting the RNA, protein, or DNA according to the conventional methods.

When the physiological saline or the buffer solution is used as the solution, in the subsequent specific steps of extracting RNA, DNA or protein, the extraction amount of the RNA extracting solution, the DNA extracting solution or the protein extracting solution may be added to the cell suspension for subsequent extraction operations. Similarly, the subsequent extraction operations may refer to the operating instructions of a reagent corresponding to the RNA extracting solution, the DNA extracting solution or the protein extracting solution, or other conventional extraction methods suitable for extraction. The extraction amount is the amount capable of extracting the RNA, DNA or protein from the cell suspension.

A ratio of the tissue sample to the solution in the step A is 10-100 mg: 100-1000 µL.

In order to facilitate the subsequent extraction procedure and enhance the extraction effect, preferably, the ratio of the tissue sample to the solution is 10-100 mg: 100-1000 µL.

When the solution is the buffer solution, the buffer solution is a PBS buffer solution.

When the solution is the buffer solution, preferably, the buffer solution is the PBS buffer solution.

The cell suspension in the present invention is the suspension in which the number of single cells accounts for more than 95% of the total number of the single cells and cell clusters after complete ablation.

### Comparative Example:

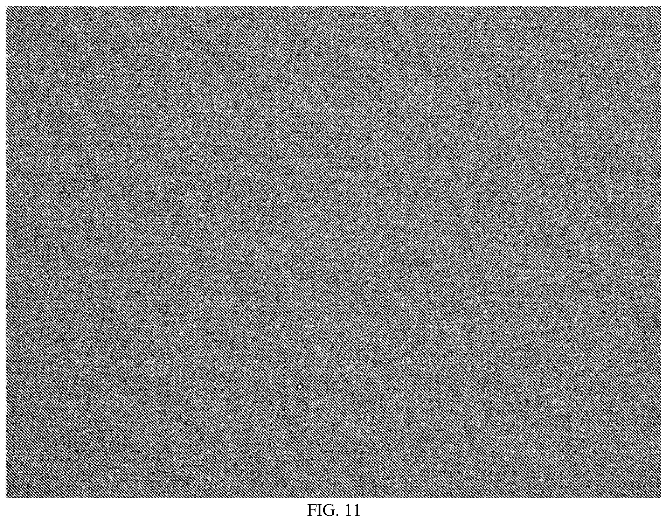

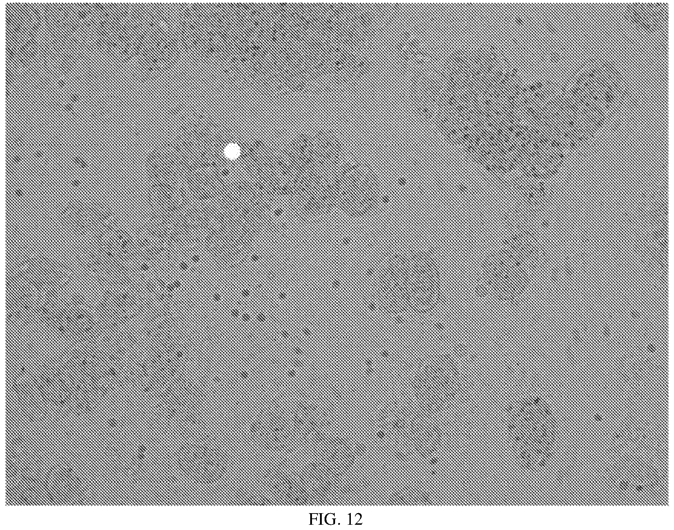

The cell suspension obtained in Embodiment 2 is shown in FIG. 11, and the tissue homogenate obtained by using a conventional tissue homogenizer is shown in FIG. 12. It can be learned from FIG. 11 and FIG. 12 that the cell suspension is obtained after the biological tissues are processed by using the method for rapidly extracting RNA, DNA or protein in the present invention, while there are a large number of cell aggregates in the tissue homogenate obtained by the existing tissue homogenizer. Obviously, compared with the conventional tissue processing methods, the method for rapidly extracting RNA, DNA or protein in the present invention has a faster processing speed and obtains a single-cell suspension.

The present invention is not limited to the above optional embodiments, and it should be understood that various changes, substitutions, and alterations could be made hereto without departing from the scope of the invention, as defined by the appended claims.

## Claims

1. An ultrasound-based handheld animal and plant tissue ablation instrument,
comprising a handheld housing (1) and an ultrasonic vibrator; wherein the housing comprises a first section and a second section, wherein the first section and the second section are disposed at an obtuse angle to each other; an end of the first section is away from the second section, and the end of the first section is the front end of the housing (1) and wherein the ultrasonic vibrator comprises an ultrasonic transducer (2);
the instrument being
**characterized in that**
the housing (1) is internally provided with a control board (4) and a drive board (5);
the ultrasonic vibrator is fixedly connected to a front end of the housing (1) and comprises an amplitude transformer (3), wherein the amplitude transformer (3) is fixedly connected to a front end of the ultrasonic transducer (2), is configured to change an amplitude of vibration input by the ultrasonic transducer (2) and to transmit out the vibration;
the control board (4) is provided with a power supply, a direct current to direct current, DCDC, conversion unit, a main control unit, a DCDC power adjustment unit and a sampling unit;
the drive board (5) is provided with a drive unit, a transformer unit and a resonance unit;
a voltage of the power supply is converted through the DCDC conversion unit and the DCDC power adjustment unit and then is supplied to the ultrasound-based handheld animal and plant tissue ablation instrument; the main control unit outputs a first pulse width modulation, PWM, signal to control the DCDC power adjustment unit to output an adjustable voltage, and outputs two second PWM signals with complementary duty cycles to the drive unit based on feedback from the sampling unit, wherein the sampling unit acquires a voltage and a current of an output circuit of the transformer unit, the transformer unit transforms the voltage, and then the resonance unit arranged in the output circuit of the transformer unit adjusts a resonance point to a resonance point of the ultrasonic vibrator, so that the ultrasonic vibrator arranged in the output circuit of the transformer unit operates in a resonance state.

2. The ultrasound-based handheld animal and plant tissue ablation instrument according to claim 1, wherein the front end of the housing (1) is provided with a connecting hole for fixedly connecting to the ultrasonic vibrator, an inner wall of the connecting hole is provided with one or more first clamping slots (103), and the ultrasonic vibrator is provided with a first step (801) matched with a first clamping slot (103) of the one or more first clamping slots (103).

3. The ultrasound-based handheld animal and plant tissue ablation instrument according to claim 2, wherein both sides of the first step (801) are provided with a first clamping block (802) and a second clamping block (803) respectively, and the first step (801) is clamped in the first clamping slot (103) through the first clamping block (802) and the second clamping block (803).

4. The ultrasound-based handheld animal and plant tissue ablation instrument according to claim 3, wherein the first clamping slot (103) is a circular clamping slot, the first step (801) is a circular step, the first clamping block (802) and the second clamping block (803) are circular clamping blocks, and the first clamping block (802) and the second clamping block (803) are sleeved on an outer wall of the ultrasonic vibrator.

5. The ultrasound-based handheld animal and plant tissue ablation instrument according to claim 2, wherein a front end of the ultrasonic vibrator is provided with the first step (801), the front end of the ultrasonic vibrator is sleeved with a first fixed cover (804), and a rear end of the first fixed cover (804) is clamped in the first clamping slot (103).

6. The ultrasound-based handheld animal and plant tissue ablation instrument according to claim 1, wherein the housing (1) comprises an upper housing (101) and a lower housing (102), wherein the upper housing (101) and the lower housing (102) are engaged with each other;
a front end of the upper housing (101) and a front end of the lower housing (102) are sleeved with a second fixed cover (805), an inner wall of the second fixed cover (805) is provided with a second step (806), and outer walls of the front end of the upper housing (101) and the front end of the lower housing (102) are provided with a second clamping slot (807) matched with the second step (806).

7. The ultrasound-based handheld animal and plant tissue ablation instrument according to claim 1, wherein a hook (9) is arranged on an outer wall of the housing (1).

8. The ultrasound-based handheld animal and plant tissue ablation instrument according to claim 1, wherein the ultrasound-based handheld animal and plant tissue ablation instrument has power of 0.5-1000 W, and ultrasonic frequency of 20-200 kHz.

9. The ultrasound-based handheld animal and plant tissue ablation instrument according to claim 1, wherein the DCDC power adjustment unit comprises:
a switch control signal receiving circuit, wherein a switch control signal receiving terminal of the switch control signal receiving circuit is connected to the main control unit to receive a switch control signal;
a first PWM signal receiving circuit, wherein a first PWM signal receiving terminal of the first PWM signal receiving circuit is connected to the main control unit to receive the first PWM signal; and
a fourth DCDC conversion unit, wherein the fourth DCDC conversion unit is configured to convert, through the switch control signal and the first PWM signal sent by the main control unit, the power supply to the adjustable voltage for output.

10. The ultrasound-based handheld animal and plant tissue ablation instrument according to claim 9, wherein the fourth DCDC conversion unit comprises a fourth voltage input filter, a fourth voltage conversion chip U1 and a fourth voltage output filter, wherein the fourth voltage input filter, the fourth voltage conversion chip U1 and the fourth voltage output filter sequentially process the voltage of the power supply;
the fourth voltage input filter comprises a filter capacitor connected to the power supply;
an upper tube drive signal reference point pin SW of the fourth voltage conversion chip U1 is connected to an adjustable voltage output terminal through an energy storage inductor L1;
voltage divider resistors R32 and R47 connected in series with the adjustable voltage output terminal sample a voltage and input the voltage to a reference voltage pin FB of the fourth voltage conversion chip U1;
the upper tube drive signal reference point pin SW of the fourth voltage conversion chip U1 is further connected to a freewheeling diode D2;
an enable pin EN is connected to a resistor R33;
a resistor timing/external clock pin RT/CLK is connected to a frequency divider resistor R85;
a frequency compensation pin COMP is connected to a capacitor C51, a capacitor C49 and a resistor R46, wherein the capacitor C51, the capacitor C49 and the resistor R46 are used to adjust a circuit and stabilize voltage output, and the capacitor C51 and the resistor R46 are connected in series and then are connected with the capacitor C49 in parallel;
the fourth voltage output filter comprises a filter capacitor connected to the adjustable voltage output terminal;
the switch control signal receiving circuit comprises a resistor R104, a resistor R105 and a transistor Q12, wherein the resistor R104 and the resistor R105 are connected in series between a converted voltage and the switch control signal receiving terminal, an emitter of the transistor Q12 is connected to the converted voltage, a base of the transistor Q12 is connected to a node between the resistor R104 and the resistor R105, and a collector of the transistor Q12 is connected to the enable pin EN of the fourth voltage conversion chip U1;
when the switch control signal is L, the transistor Q12 is turned on, and the fourth voltage conversion chip U1 is activated to work;
the first PWM signal receiving circuit comprises an RC filter and a first voltage follower U3B, wherein the RC filter and the first voltage follower U3B sequentially process the first PWM signal;
the first PWM signal received by the first PWM signal receiving terminal is filtered through the RC filter and then input to a non-inverting input terminal of the first voltage follower U3B, and an output terminal of the first voltage follower U3B is connected to a node between the voltage divider resistors R32 and R47 through a voltage divider resistor R54.

11. The ultrasound-based handheld animal and plant tissue ablation instrument according to claim 1, wherein the drive unit comprises a first drive unit and a second drive unit;
a second PWM signal receiving terminal N of the first drive unit receives a first-channel second PWM signal sent by the main control unit, and an output terminal of the first drive unit is connected to a dotted terminal of a primary coil of the transformer unit;
a second PWM signal receiving terminal P of the second drive unit receives a second-channel second PWM signal sent by the main control unit, and an output terminal of the second drive unit is connected to a non-dotted terminal of the primary coil of the transformer unit;
the first-channel second PWM signal received by the second PWM signal receiving terminal N controls a first drive metal-oxide-semiconductor, MOS, transistor Q6 to be on/off, and the second-channel second PWM signal received by the second PWM signal receiving terminal P controls a second drive MOS transistor Q2 to be off/on.

12. The ultrasound-based handheld animal and plant tissue ablation instrument according to claim 11, wherein the transformer unit is a push-pull transformer, and the adjustable voltage output terminal of the DCDC power adjustment unit is connected to a non-dotted terminal of a first primary coil and a dotted terminal of a second primary coil of the push-pull transformer;
the first drive unit comprises resistors R10 and R14 and the first drive MOS transistor Q6, wherein the resistors R10 and R14 are connected in series between the ground and the second PWM signal receiving terminal N, a gate of the first drive MOS transistor Q6 is connected to a node between the resistors R10 and R14, a source of the first drive MOS transistor Q6 is grounded, and a drain of the first drive MOS transistor Q6 is the output terminal of the first drive unit and is connected to a dotted terminal of the first primary coil of the push-pull transformer; and
the second drive unit comprises resistors R5 and R13 and the second drive MOS transistor Q2, wherein the resistors R5 and R13 are connected in series between the ground and the second PWM signal receiving terminal P, a gate of the second drive MOS transistor Q2 is connected to a node between the resistors R5 and R13, a source of the second drive MOS transistor Q2 is grounded, and a drain of the second drive MOS transistor Q2 is the output terminal of the second drive unit and is connected to a non-dotted terminal of the second primary coil of the push-pull transformer.

13. The ultrasound-based handheld animal and plant tissue ablation instrument according to claim 1, wherein the resonance unit is a series LC resonator, and the series LC resonator comprises an inductor T1 and a capacitor C1, wherein the inductor T1 and the capacitor C1 are connected in series in the output circuit of the transformer unit.

14. The ultrasound-based handheld animal and plant tissue ablation instrument according to claim 1, wherein the sampling unit comprises a voltage sampling unit for acquiring a voltage of the output circuit of the transformer unit and a current sampling unit for acquiring a current of the output circuit of the transformer unit, and the output circuit of the transformer unit is provided with a plurality of sampling resistors connected in series;
a voltage signal is acquired by a voltage sampling terminal connected to a node between the plurality of sampling resistors, is sent to the voltage sampling unit for filtering and amplification, and then is sent to the main control unit for resonance adjustment;
a current signal is acquired by a current sampling terminal connected in series to the output circuit of the transformer unit, is sent to the current sampling unit for filtering and amplification, and then is sent to the main control unit for resonance adjustment.

15. The ultrasound-based handheld animal and plant tissue ablation instrument according to claim 1, wherein the main control unit comprises a main control chip U2 and a main control chip peripheral circuit;
the main control chip U2 outputs the first PWM signal to control the DCDC power adjustment unit to output the adjustable voltage, and outputs the two second PWM signals with the complementary duty cycles to the drive unit based on the feedback from the sampling unit, wherein the sampling unit acquires the voltage and the current of the output circuit of the transformer unit.

16. The ultrasound-based handheld animal and plant tissue ablation instrument according to claim 15, wherein the main control unit further comprises an auxiliary chip U1 and an auxiliary chip peripheral circuit;
the main control chip U2 of the main control unit sends an instruction to the auxiliary chip U1 based on the feedback from the sampling unit, wherein the sampling unit acquires the voltage and the current of the output circuit of the transformer unit;
the auxiliary chip U1 receives the instruction from the main control chip U2 and outputs the two second PWM signals with the complementary duty cycles to the drive unit, wherein the two second PWM signals with the complementary duty cycles are further fed back to the main control chip U2.

17. The ultrasound-based handheld animal and plant tissue ablation instrument according to claim 15, wherein the two second PWM signals with the complementary duty cycles are further amplified by a first signal amplifying output circuit and a second signal amplifying output circuit respectively and then are sent to the drive unit.

18. The ultrasound-based handheld animal and plant tissue ablation instrument according to claim 15, further comprising a display unit, a button (6), a charging interface, a memory, a USB interface unit and/or a touch unit connected to the main control chip U2; wherein when the main control chip U2 is connected to the button and the display unit, the button (6) and a display screen (7) of the display unit are arranged on a surface of the second section of the housing (1).

## Patentansprüche

1. Ultraschallbasiertes Tier- und Pflanzengewebeablationshandinstrument, umfassend ein mit der Hand gehaltenes Gehäuse (1) und einen Ultraschallvibrator; wobei das Gehäuse einen ersten Abschnitt und einen zweiten Abschnitt umfasst, wobei der erste Abschnitt und der zweite Abschnitt in einem stumpfen Winkel zueinander angeordnet sind; ein Ende des ersten Abschnitts von dem zweiten Abschnitt entfernt ist und das Ende des ersten Abschnitts das Vorderende des Gehäuses (1) ist, und wobei der Ultraschallvibrator einen Ultraschallmessgeber (2) umfasst; wobei da Instrument **dadurch gekennzeichnet ist, dass**
das Gehäuse (1) im Inneren mit einer Steuerplatine (4) und einer Ansteuerungsplatine (5) versehen ist;
der Ultraschallvibrator fest mit einem Vorderende des Gehäuses (1) verbunden ist und ein Amplitudentransformationsstück (3) umfasst, wobei das Amplitudentransformationsstück (3) fest mit einem Vorderende des Ultraschallmessgebers (2) verbunden ist und dazu konfiguriert ist, eine Amplitude der durch den Ultraschallmessgeber (2) eingegebenen Vibration zu ändern und die Vibration nach außen zu übertragen;
die Steuerplatine (4) mit einer Leistungsversorgung, einer Gleichstrom-Gleichstrom(DCDC)-Umwandlungseinheit, einer Hauptsteuerungseinheit, einer DCDC-Leistungseinstelleinheit und einer Abtasteinheit versehen ist;
die Ansteuerungsplatine (5) mit einer Ansteuerungseinheit, einer Transformatoreinheit und einer Resonanzeinheit versehen ist;
eine Spannung der Leistungsversorgung durch die DCDC-Umwandlungseinheit und die DCDC-Leistungseinstelleinheit umgewandelt wird und dann dem ultraschallbasierten Tier- und Pflanzengewebeablationshandinstrument zugeführt wird; die Hauptsteuerungseinheit ein erstes Impulsbreitenmodulations(PWM)-Signal ausgibt, um die DCDC-Leistungseinstelleinheit zum Ausgeben einer einstellbaren Spannung zu steuern, und auf Grundlage von Rückkopplung von der Abtasteinheit zwei zweite PWM-Signale mit komplementären Abtastraten an die Ansteuerungseinheit ausgibt, wobei die Abtasteinheit eine Spannung und einen Strom einer Ausgabeschaltung der Transformatoreinheit erfasst, die Transformatoreinheit die Spannung umsetzt und die Resonanzeinheit, die in der Ausgabeschaltung der Transformatoreinheit angeordnet ist, dann einen Resonanzpunkt auf einen Resonanzpunkt des Ultraschallvibrators einstellt, sodass der Ultraschallvibrator, der in der Ausgabeschaltung der Transformatoreinheit angeordnet ist, in einem Resonanzzustand arbeitet.

2. Ultraschallbasiertes Tier- und Pflanzengewebeablationshandinstrument nach Anspruch 1, wobei das Vorderende des Gehäuses (1) mit einem Verbindungsloch zum festen Verbinden mit dem Ultraschallvibrator versehen ist, eine Innenwand des Verbindungslochs mit einem oder mehreren ersten Klemmschlitzen (103) versehen ist und der Ultraschallvibrator mit einer ersten Stufe (801) versehen ist, die mit einen ersten Klemmschlitz (103) von dem einen oder den mehreren ersten Klemmschlitzen (103) übereinstimmt.

3. Ultraschallbasiertes Tier- und Pflanzengewebeablationshandinstrument nach Anspruch 2, wobei die beiden Seiten der ersten Stufe (801) jeweils mit einem ersten Klemmblock (802) bzw. einem zweiten Klemmblock (803) versehen sind und die erste Stufe (801) durch den ersten Klemmblock (802) und den zweiten Klemmblock (803) in den ersten Klemmschlitz (103) geklemmt ist.

4. Ultraschallbasiertes Tier- und Pflanzengewebeablationshandinstrument nach Anspruch 3, wobei der erste Klemmschlitz (103) ein kreisförmiger Klemmschlitz ist, die erste Stufe (801) eine kreisförmige Stufe ist, der erste Klemmblock (802) und der zweite Klemmblock (803) kreisförmige Klemmblöcke sind und der erste Klemmblock (802) und der zweite Klemmblock (803) auf eine Außenwand des Ultraschallvibrators gesetzt sind.

5. Ultraschallbasiertes Tier- und Pflanzengewebeablationshandinstrument nach Anspruch 2, wobei ein Vorderende des Ultraschallvibrator mit der ersten Stufe (801) versehen ist, eine erste feste Abdeckung (804) auf das Vorderende des Ultraschallvibrators gesetzt ist und ein hinteres Ende der ersten festen Abdeckung (804) in den ersten Klemmschlitz (103) geklemmt ist.

6. Ultraschallbasiertes Tier- und Pflanzengewebeablationshandinstrument nach Anspruch 1, wobei das Gehäuse (1) ein oberes Gehäuse (101) und ein unteres Gehäuse (102) umfasst, wobei das obere Gehäuse (101) und das untere Gehäuse (102) miteinander in Eingriff stehen;
ein zweite feste Abdeckung (805) auf ein Vorderende des oberen Gehäuses (101) und ein Vorderende des unteren Gehäuses (102) gesetzt ist, eine Innenwand der zweiten festen Abdeckung (805) mit einen zweiten Stufe (806) versehen ist, und Außenwände des Vorderendes des oberen Gehäuses (101) und des Vorderendes des unteren Gehäuses (102) mit einem zweiten Klemmschlitz (807) versehen sind, der mit der zweiten Stufe (806) übereinstimmt.

7. Ultraschallbasiertes Tier- und Pflanzengewebeablationshandinstrument nach Anspruch 1, wobei ein Haken (9) an einer Außenwand des Gehäuses (1) angeordnet ist.

8. Ultraschallbasiertes Tier- und Pflanzengewebeablationshandinstrument nach Anspruch 1, wobei das ultraschallbasiertes Tier- und Pflanzengewebeablationshandinstrument eine Leistung von 0,5-1000 W und eine Ultraschallfrequenz von 20-200 kHz aufweist.

9. Ultraschallbasiertes Tier- und Pflanzengewebeablationshandinstrument nach Anspruch 1, wobei die DCDC-Leistungseinstelleinheit umfasst:
eine Schaltsteuerungssignalempfangsschaltung, wobei ein Schaltsteuerungssignalempfangsanschluss der Schaltsteuerungssignalempfangsschaltung mit der Hauptsteuerungseinheit verbunden ist, um ein Schaltsteuerungssignal zu empfangen;
eine erste PWM-Signalempfangseinheit, wobei ein erster PWM-Signalempfangsanschluss der ersten PWM-Signalempfangseinheit mit der Hauptsteuerungseinheit verbunden ist, um das erste PWM-Signal zu empfangen; und
eine vierte DCDC-Umwandlungseinheit, wobei die vierte DCDC-Umwandlungseinheit dazu konfiguriert ist, durch das Schaltsteuerungssignal und das erste PWM-Signal von der Hauptsteuerungseinheit die Leistungsversorgung auf die einstellbare Spannung zur Ausgabe einzustellen.

10. Ultraschallbasiertes Tier- und Pflanzengewebeablationshandinstrument nach Anspruch 9, wobei die vierte DCDC-Umwandlungseinheit ein viertes Spannungseingangsfilter, einen vierten Spannungsumwandlungschip U1 und ein viertes Spannungsausgangsfilter umfasst, wobei das vierte Spannungseingangsfilter, der vierte Spannungsumwandlungschip U1 und das vierte Spannungsausgangsfilter die Spannung der Leistungsversorgung der Reihe nach verarbeiten;
das vierte Spannungseingangsfilter einen Filterkondensator umfasst, der mit der Leistungsversorgung verbunden ist;
ein oberer Rohransteuerungssignalbezugspunktpin SW des vierten Spannungsumwandlungschips U1 durch einen Energiespeicherungsinduktor L1 mit einem einstellbaren Spannungsausgangsanschluss verbunden ist;
Spannungsteilungswiderstände R32 und R47, die mit dem einstellbaren Spannungsausgangsanschluss in Reihe geschaltet sind, eine Spannung abtasten und die Spannung an einen Referenzspannungspin FB des vierten Spannungsumwandlungschips U1 anlegen;
der obere Rohransteuerungssignalbezugspunktpin SW des vierten Spannungsumwandlungschip U1 ferner mit einer Freilaufdiode D2 verbunden ist;
ein Aktivierungspin EN mit einem Widerstand R33 verbunden ist;
ein Widerstandstaktungs-/Externer-Taktgeber-Pin RT/CLK mit einem Frequenzteilungswiderstand R85 verbunden ist;
ein Frequenzausgleichspin COMP mit einem Kondensator C51, einem Kondensator C49 und einem Widerstand R46 verbunden ist, wobei der Kondensator C51, der Kondensator C49 und der Widerstand R46 dazu dienen, einen Schaltkreis einzustellen und einen Spannungsausgang zu stabilisieren, und der Kondensator C51 und der Widerstand R46 in Reihe geschaltet sind und dann parallel mit dem Kondensator C49 geschaltet sind;
das vierte Spannungsausgangsfilter einen Filterkondensator umfasst, der mit dem einstellbaren Spannungsausgangsanschluss verbunden ist;
die Schaltsteuerungssignalempfangsschaltung einen Widerstand R104, einen Widerstand R105 und einen Transistor Q12 umfasst, wobei der Widerstand R104 und der Widerstand R105 zwischen einer umgewandelten Spannung und dem Schaltsteuerungssignalempfangsanschluss in Reihe geschaltet sind, ein Emitter des Transistors Q12 mit der umgewandelten Spannung verbunden ist, eine Base des Transistors Q12 mit einem Knoten zwischen dem Widerstand R104 und dem Widerstand R105 verbunden ist und ein Kollektor des Transistors Q12 mit dem Aktivierungspin EN des vierten Spannungsumwandlungschips U1 verbunden ist;
wobei, wenn das Schaltsteuerungssignal L ist, der Transistor Q12 angeschaltet wird und der vierte Spannungsumwandlungschip U1 aktiviert wird;
wobei die erste PWM-Signalempfangseinheit ein RC-Filter und eine erste Spannungsverfolgungseinrichtung U3B umfasst, wobei das RC-Filter und die erste Spannungsverfolgungseinrichtung U3B nacheinander das erste PWM-Signal verarbeiten;
wobei das erste PWM-Signal, das durch den ersten PWM-Signalempfangsanschluss empfangen wird, durch das RC-Filter gefiltert und dann in einen nicht invertierenden Eingangsanschluss der ersten Spannungsverfolgungseinrichtung U3B eingespeist wird, und ein Ausgangsanschluss der ersten Spannungsverfolgungseinrichtung U3B durch einen Spannungsteilungswiderstand R54 mit einem Knoten zwischen den Spannungsteilungswiderständen R32 und R47 verbunden ist.

11. Ultraschallbasiertes Tier- und Pflanzengewebeablationshandinstrument nach Anspruch 1, wobei die Ansteuerungseinheit einen erste Ansteuerungseinheit und eine zweite Ansteuerungseinheit umfasst;
ein zweiter PWM-Signalempfangsanschluss N der ersten Ansteuerungseinheit ein zweites PWM-Signal des ersten Kanals von der Hauptsteuerungseinheit empfängt und ein Ausgangsanschluss der ersten Ansteuerungseinheit mit einem punktierten Anschluss einer Primärspule der Transformatoreinheit verbunden ist;
ein zweiter PWM-Signalempfangsanschluss P der zweiten Ansteuerungseinheit ein zweites PWM-Signal des zweiten Kanals von der Hauptsteuerungseinheit empfängt und ein Ausgangsanschluss der zweiten Ansteuerungseinheit mit einem nichtpunktierten Anschluss der Primärspule der Transformatoreinheit verbunden ist;
das zweite PWM-Signal des ersten Kanals, das durch den zweiten PWM-Signalempfangsanschluss N empfangen wird, das An-/Ausschalten eines ersten Ansteuerungs-Metalloxidhalbleiter(MOS)-Transistors Q6 steuert und das zweite PWM-Signal des zweiten Kanals, das durch den zweiten PWM-Signalempfangsanschluss P empfangen wird, das Aus-/Anschalten eines zweiten Ansteuerungs-MOS-Transistors Q2 steuert.

12. Ultraschallbasiertes Tier- und Pflanzengewebeablationshandinstrument nach Anspruch 11, wobei die Transformatoreinheit ein Push-Pull-Transformator ist und der einstellbaren Spannungsausgangsanschluss der DCDC-Leistungseinstelleinheit mit einem nichtpunktierten Anschluss einer ersten Primärspule und einem punktierten Anschluss einer zweiten Primärspule des Push-Pull-Transformator verbunden ist;
die erste Ansteuerungseinheit Widerstände R10 und R14 und den ersten Ansteuerungs-MOS-Transistor Q6 umfasst, wobei die Widerstände R10 und R14 zwischen der Masse und dem zweiten PWM-Signalempfangsanschluss N in Reihe geschaltet sind, ein Gate des ersten Ansteuerungs-MOS-Transistors Q6 mit einem Knoten zwischen den Widerständen R10 und R14 verbunden ist, eine Source des ersten Ansteuerungs-MOS-Transistors Q6 mit Masse verbunden ist und ein Drain des ersten Ansteuerungs-MOS-Transistors Q6 der Ausgangsanschluss der ersten Ansteuerungseinheit ist und mit einem punktierten Anschluss der ersten Primärspule des Push-Pull-Transformators verbunden ist; und
die zweite Ansteuerungseinheit Widerstände R5 und R13 und den zweiten Ansteuerungs-MOS-Transistor Q2 umfasst, wobei die Widerstände R5 und R13 zwischen der Masse und dem zweiten PWM-Signalempfangsanschluss P in Reihe geschaltet sind, ein Gate des zweiten Ansteuerungs-MOS-Transistors Q2 mit einem Knoten zwischen den Widerständen R5 und R13 verbunden ist, eine Source des zweiten Ansteuerungs-MOS-Transistors Q2 mit Masse verbunden ist und ein Drain des zweiten Ansteuerungs-MOS-Transistors Q2 der Ausgangsanschluss der zweiten Ansteuerungseinheit ist und mit einem punktierten Anschluss der zweiten Primärspule des Push-Pull-Transformators verbunden ist.

13. Ultraschallbasiertes Tier- und Pflanzengewebeablationshandinstrument nach Anspruch 1, wobei die Resonanzeinheit ein Reihen-LC-Resonator ist und der Reihen-LC-Resonator einen Induktor T1 und einen Kondensator C1 umfasst, wobei der Induktor T1 und der Kondensator C1 in der Ausgabeschaltung der Transformatoreinheit in Reihe geschaltet sind.

14. Ultraschallbasiertes Tier- und Pflanzengewebeablationshandinstrument nach Anspruch 1, wobei die Abtasteinheit eine Spannungsabtasteinheit zum Erfassen einer Spannung der Ausgabeschaltung der Transformatoreinheit und eine Stromabtasteinheit zum Erfassen eines Stroms der Ausgabeschaltung der Transformatoreinheit umfasst, und die Ausgabeschaltung der Transformatoreinheit mit einer Vielzahl von Abtastwiderständen versehen ist, die in Reihe geschaltet sind;
ein Spannungssignal durch einen Spannungsabtastanschluss erfasst wird, der mit einem Knoten zwischen der Vielzahl von Abtastwiderständen verbunden ist, zur Filterung und Verstärkung an die Spannungsabtasteinheit gesendet wird und dann zur Resonanzeinstellung an die Hauptsteuerungseinheit gesendet wird;
ein Stromsignal durch einen Stromabtastanschluss erfasst wird, der mit der Ausgabeschaltung der Transformatoreinheit in Reihe geschaltet ist, zur Filterung und Verstärkung an die Stromabtasteinheit gesendet wird und dann zur Resonanzeinstellung an die Hauptsteuerungseinheit gesendet wird.

15. Ultraschallbasiertes Tier- und Pflanzengewebeablationshandinstrument nach Anspruch 1, wobei die Hauptsteuerungseinheit einen Hauptsteuerchip U2 und eine Hauptsteuerchipperipherieschaltung umfasst;
der Hauptsteuerchip U2 das erste PWM-Signal ausgibt, um die DCDC-Leistungseinstelleinheit zum Ausgeben der einstellbaren Spannung zu steuern, und die zwei zweiten PWM-Signale mit den komplementären Abtastraten auf Grundlage von Rückkopplung von der Abtasteinheit an die Ansteuerungseinheit ausgibt, wobei die Abtasteinheit die Spannung und den Strom der Ausgabeschaltung der Transformatoreinheit erfasst.

16. Ultraschallbasiertes Tier- und Pflanzengewebeablationshandinstrument nach Anspruch 15, wobei die Hauptsteuerungseinheit ferner einen Hilfschip U1 und eine Hilfschipperipherieschaltung umfasst;
der Hauptsteuerchip U2 der Hauptsteuerungseinheit auf Grundlage der Rückkopplung von der Abtasteinheit eine Anweisung an den Hilfschip U1 sendet, wobei die Abtasteinheit die Spannung und den Strom der Ausgabeschaltung der Transformatoreinheit erfasst;
der Hilfschip U1 die Anweisung von dem Hauptsteuerchip U2 empfängt und die zwei zweiten PWM-Signale mit den komplementären Abtastraten an die Ansteuerungseinheit ausgibt, wobei die zwei zweiten PWM-Signale mit den komplementären Abtastraten weiter an den Hauptsteuerchip U2 rückgekoppelt werden.

17. Ultraschallbasiertes Tier- und Pflanzengewebeablationshandinstrument nach Anspruch 15, wobei die zwei zweiten PWM-Signale mit den komplementären Abtastraten jeweils weiter durch eine erste Signalverstärkungsausgabeschaltung und eine zweite Signalverstärkungsausgabeschaltung verstärkt werden und dann an die Ansteuerungseinheit gesendet werden.

18. Ultraschallbasiertes Tier- und Pflanzengewebeablationshandinstrument nach Anspruch 15, ferner umfassend eine Anzeigeeinheit, eine Taste (6), eine Ladeschnittstelle, einen Speicher, eine USB-Schnittstelleneinheit und/oder eine Berührungseinheit, die mit dem Hauptsteuerchip U2 verbunden ist; wobei, wenn der Hauptsteuerchip U2 mit der Taste und der Anzeigeeinheit verbunden ist, die Taste (6) und ein Anzeigebildschirm (7) der Anzeigeeinheit an einer Fläche des zweiten Abschnitts des Gehäuses (1) angeordnet sind.

## Revendications

1. Un instrument portable d'ablation de tissus animaux et végétaux basé sur les ultrasons, comprenant un logement portable (1) et un oscillateur à ultrasons ; dans lequel le logement comprend une première section et une deuxième section, dans lequel la première section et la deuxième section sont agencées en angle obtus l'une par rapport à l'autre ; une terminaison de la première section est distale de la deuxième section, et la terminaison de la première section constitue la terminaison avant du logement (1) et dans lequel l'oscillateur à ultrasons comprend un transducteur ultrasonique (2) ; l'instrument étant **caractérisé en ce que**
le logement (1) est muni intérieurement d'un panneau de commande (4) et d'une carte de pilotage (5) ;
l'oscillateur à ultrasons est raccordé fixement à une terminaison avant du logement (1) et comprend un transformateur d'amplitude (3), dans lequel le transformateur d'amplitude (3) est raccordé fixement à une terminaison avant du transducteur ultrasonique (2), et est configuré pour modifier l'amplitude de vibration fournie par le transducteur ultrasonique (2) et pour transmettre les vibrations ;
le panneau de commande (4) est muni d'une alimentation électrique, de courant continu à courant continu, DCDC, une unité de conversion, une unité de commande principale, une unité de réglage électrique DCDC et une unité d'échantillonnage ;
la carte de pilotage (5) est munie d'une unité de pilotage, d'une unité de transformateur et d'une unité de résonance ;
la tension de l'alimentation électrique est converti via l'unité de conversion DCDC et l'unité de réglage électrique DCDC et est ensuite fournie à l'instrument portable d'ablation de tissus animaux et végétaux basé sur les ultrasons ; l'unité de commande principale produit une première modulation de largeur d'impulsion, PWM, un signal pour contrôler l'unité de réglage électrique DCDC afin de produire une tension réglable, et produit deux deuxième signaux PWM avec cycles de service complémentaire à l'unité de pilotage basés sur le retour de l'unité d'échantillonnage, dans lequel l'unité d'échantillonnage acquière la tension et le courant de circuit de sortie de l'unité de transformateur, l'unité de transformateur transforme la tension, puis l'unité de résonance agencée dans le circuit de sortie de l'unité de transformateur ajuste un point de résonance au point de résonance de l'oscillateur à ultrasons, de sorte que l'oscillateur à ultrasons agencé dans le circuit de sortie de l'unité de transformateur fonctionne en état de résonance.

2. Un instrument portable d'ablation de tissus animaux et végétaux basé sur les ultrasons selon la revendication 1, dans lequel la terminaison avant du logement (1) est munie d'un trou de raccordement afin de raccorder fixement l'oscillateur à ultrasons, une paroi interne du trou de raccordement est munie d'une ou de plusieurs premières fentes de serrage (103), et l'oscillateur à ultrasons est muni d'une première marche (801) correspondant à la première fente de serrage (103) d'une ou de plusieurs premières fentes de serrage (103).

3. Un instrument portable d'ablation de tissus animaux et végétaux basé sur les ultrasons selon la revendication 2, dans lequel les deux côtés de la première marche (801) sont munis d'un premier bloc de serrage (802) et d'un deuxième bloc de serrage (803) respectivement, et la première marche (801) est serrée dans la première fente de serrage (103) via le premier bloc de serrage (802) et le deuxième bloc de serrage (803).

4. Un instrument portable d'ablation de tissus animaux et végétaux basé sur les ultrasons selon la revendication 3, dans lequel la première fente de serrage (103) est une fente de serrage circulaire, la première marche (801) est une marche circulaire, le premier bloc de serrage (802) et le deuxième bloc de serrage (803) sont des blocs de serrage circulaires, et le premier bloc de serrage (802) et le deuxième bloc de serrage (803) sont emmanchés sur une paroi externe de l'oscillateur à ultrasons.

5. Un instrument portable d'ablation de tissus animaux et végétaux basé sur les ultrasons selon la revendication 2, dans lequel une terminaison avant de l'oscillateur à ultrasons est munie d'une première marche (801), la terminaison avant de l'oscillateur à ultrasons est emmanchée par un premier couvercle fixe (804), et une terminaison arrière du premier couvercle fixe (804) est serrée dans la première fente de serrage (103).

6. Un instrument portable d'ablation de tissus animaux et végétaux basé sur les ultrasons selon la revendication 1, dans lequel le logement (1) comprend un logement supérieur (101) et un logement inférieur (102), dans lesquels le logement supérieur (101) et le logement inférieur (102) sont mutuellement engagés ;
une terminaison avant du logement supérieur (101) et une terminaison avant du logement inférieur (102) sont emmanchées par un deuxième couvercle fixe (805), une paroi interne du deuxième couvercle fixe (805) est munie d'une deuxième marche (806), et les parois externe de la terminaison avant du logement supérieur (101) et la terminaison avant du logement inférieur (102) sont munies d'une deuxième fente de serrage (807) correspondant à la deuxième marche (806).

7. Un instrument portable d'ablation de tissus animaux et végétaux basé sur les ultrasons selon la revendication 1, dans lequel un crochet (9) est agencé sur la paroi externe du logement (1).

8. Un instrument portable d'ablation de tissus animaux et végétaux basé sur les ultrasons selon la revendication 1, dans lequel l'instrument portable d'ablation de tissus animaux et végétaux basé sur les ultrasons dispose d'une puissance de 0,5-1000 W, et d'une fréquence ultrasonique de 20-200 kHz.

9. Un instrument portable d'ablation de tissus animaux et végétaux basé sur les ultrasons selon la revendication 1, dans lequel l'unité de réglage électrique DCDC comprend :
un circuit de réception de signal de commande de commutation, dans lequel une borne de réception de signal de commande de commutation du circuit de réception de signal de commande de commutation est raccordée à l'unité de commande principale pour recevoir un signal de commande de commutation ;
un circuit de réception de premier signal PWM, dans lequel une borne de réception de premier signal PWM du circuit de réception de premier signal PWM est raccordée à l'unité de commande principale pour recevoir le premier signal PWM ; et
une quatrième unité de conversion DCDC, dans laquelle la quatrième unité de conversion DCDC est configurée pour convertir, via le signal de commande de commutation et le premier signal PWM envoyé par l'unité de commande principale, l'alimentation électrique à la tension ajustable de sortie.

10. Un instrument portable d'ablation de tissus animaux et végétaux basé sur les ultrasons selon la revendication 9, dans lequel la quatrième unité de conversion DCDC comprend un quatrième filtre d'entrée de tension, une quatrième puce U1 de conversion de tension et un quatrième filtre de sortie de tension, dans lesquels le quatrième filtre d'entrée de tension, la quatrième puce U1 de conversion de tension et le quatrième filtre de sortie de tension traitent de façon séquentielle la tension de l'alimentation électrique ;
le quatrième filtre d'entrée de tension comprend un condensateur de filtre raccordé à l'alimentation électrique ;
la broche SW du point de référence de signal de pilotage de tube supérieur de la quatrième puce U1 de conversion de tension est raccordée à une borne de sortie de tension réglable via un inducteur de stockage d'énergie L1 ;
les résistances de diviseur de tension R32 et R47 raccordées en série à la borne de sortie de tension réglable échantillonnent une tension et entrent la tension sur une broche de tension de référence FB de la quatrième puce U1 de conversion de tension ;
la broche SW du point de référence de signal de pilotage de tube supérieur de la quatrième puce U1 de conversion de tension est en outre raccordée à une diode de roue libre D2 ;
une broche d'activation EN est raccordée à une résistance R33 ;
une broche de d'horloge externe/synchronisation de résistance RT/CLK est raccordée à une résistance de diviseur de fréquence R85 ;
une broche de compensation de fréquence COMP est raccordée à un condensateur C51, un condensateur C49 et une résistance R46, dans lesquels le condensateur C51, le condensateur C49 et la résistance R46 sont utilisés pour ajuster un circuit et stabiliser la sortie de tension, et le condensateur C51 et la résistance R46 sont raccordés en série puis sont raccordés au condensateur C49 en parallèle ;
le quatrième filtre de sortie de tension comprend un condensateur de filtre raccordé à la borne de sortie de tension réglable ;
le circuit de réception de signal de commande de commutation comprend une résistance R104, une résistance R105 et un transistor Q12, dans lesquel la résistance R104 et la résistance R105 sont raccordées en série entre une tension convertie et le circuit de réception de signal de commande de commutation, un émetteur du transistor Q12 est raccordé à la tension convertie, une base du transistor Q12 est raccordée à un nœud entre la résistance R104 et la résistance R105, et un collecteur du transistor Q12 est raccordé à la broche d'activation EN de la quatrième puce U1 de conversion de tension ;
lorsque le signal de commande de commutation est L, le transistor Q12 est mis en marche, et le fonctionnement de la quatrième puce U1 de conversion de tension est activé ;
le circuit de réception de premier signal PWM comprend un filtre RC et un premier suiveur de tension U3B, dans lesquels le filtre RC et le premier suiveur de tension U3B traitent en séquence le premier signal PWM ;
le premier signal PWM reçu par la borne de réception de premier signal PWM est filtré par le filtre RC puis entré dans une borne d'entrée non-inverseuse du premier suiveur de tension U3B, et une borne de sortie du premier suiveur de tension U3B est raccordée à un nœud entre les résistances de diviseur de tension R32 et R47 via une résistance de diviseur de tension R54.

11. Un instrument portable d'ablation de tissus animaux et végétaux basé sur les ultrasons selon la revendication 1, dans lequel l'unité de pilotage comprend une unité de premier pilotage et une unité de deuxième pilotage ;
une borne N de réception de deuxième signal PWM de l'unité de premier pilotage reçoit un deuxième signal PWM de premier canal envoyé par l'unité de commande principale, et une borne de sortie de l'unité de premier pilotage est raccordée à une borne pointillée d'une bobine primaire de l'unité de transformateur ;
une borne P de réception de deuxième signal PWM de l'unité de deuxième pilotage reçoit un deuxième signal PWM de deuxième canal envoyé par l'unité de commande principale, et une borne de sortie de l'unité de deuxième pilotage est raccordée à une borne non-pointillée de la bobine primaire de l'unité de transformateur ;
le deuxième signal PWM de deuxième canal reçu par la borne N de réception de deuxième signal PWM commande la mise en marche/arrêt du transistor Q6 semi-conducteur métal oxyde, MOS, de premier pilotage et le deuxième signal PWM de deuxième canal reçu par la borne P de réception de deuxième signal PWM commande la mise en marche/arrêt du transistor Q2 MOS de deuxième pilotage.

12. Un instrument portable d'ablation de tissus animaux et végétaux basé sur les ultrasons selon la revendication 11, dans lequel l'unité de transformateur est un transformateur symétrique, et la borne de sortie de tension réglable de l'unité de réglage électrique DCDC est raccordée à une borne non-pointillée d'une première bobine primaire et à une borne pointillée d'une deuxième bobine primaire du transformateur symétrique ;
l'unité de premier pilotage comprend les résistances R10 et R14 et le transistor Q6 MOS de premier pilotage, dans lesquels les résistances R10 et R14 sont raccordées en série entre la terre et la borne N de réception de deuxième signal PWM, une grille du transistor Q6 MOS de premier pilotage est raccordée à un nœud entre les résistances R10 et R14, la source du transistor Q6 MOS de premier pilotage est mise à la terre, et le drain du transistor Q6 MOS de premier pilotage est la sortie de la borne de l'unité de premier pilotage et est raccordé à une borne pointillée de la première bobine primaire du transformateur symétrique ; et
l'unité de deuxième pilotage comprend les résistances R5 et R13 et le transistor MOS Q2 de deuxième pilotage, dans laquelle les résistances R5 et R13 sont raccordées en série entre la terre et la borne P de réception de deuxième signal PWM, une grille de transistor MOS Q2 de deuxième pilotage est raccordée à un nœud entre les résistances R5 et R13, la source du transistor MOS Q2 de deuxième pilotage est mise à la terre, et le drain du transistor MOS Q2 de deuxième pilotage est la sortie de la borne de l'unité de deuxième pilotage et est raccordé à une borne non-pointillée de la deuxième bobine primaire du transformateur symétrique.

13. Un instrument portable d'ablation de tissus animaux et végétaux basé sur les ultrasons selon la revendication 1, dans lequel l'unité de résonance est un résonateur LC en série, et le résonateur LC en série comprend un inducteur T1 et un condensateur C1, dans lequel l'inducteur T1 et le condensateur C1 sont raccordés en série sur le circuit de sortie de l'unité de transformateur.

14. Un instrument portable d'ablation de tissus animaux et végétaux basé sur les ultrasons selon la revendication 1, dans lequel l'unité d'échantillonnage comprend une unité d'échantillonnage de tension pour acquérir la tension du circuit de sortie de l'unité de transformateur et une unité d'échantillonnage de courant pour acquérir le courant du circuit de sortie de l'unité de transformateur, et le circuit de sortie de l'unité de transformateur est munie d'une pluralité de résistances d'échantillonnage raccordées en série ;
un signal de tension acquis par une borne d'échantillonnage de tension raccordée à un nœud entre la pluralité de résistances d'échantillonnage, est envoyé à l'unité d'échantillonnage de tension pour filtrage et amplification, puis est envoyé à l'unité de commande principale pour réglage de résonance ;
un signal de courant acquis par la borne d'échantillonnage de courant raccordée en série au circuit de sortie de l'unité de transformateur, est envoyé à l'unité d'échantillonnage de courant pour filtrage et amplification, puis est envoyé à l'unité de commande principale pour réglage de résonance.

15. Un instrument portable d'ablation de tissus animaux et végétaux basé sur les ultrasons selon la revendication 1, dans lequel l'unité de commande principale comprend une puce U2 de commande principale et un circuit périphérique de puce de commande principale ;
la puce U2 de commande principale produit le premier signal PWM pour commander à l'unité de réglage électrique DCDC de produire une tension réglable, et produit les deux deuxième signaux PWM avec cycles de service complémentaire à l'unité de pilotage basée sur le retour de l'unité d'échantillonnage, dans laquelle l'unité d'échantillonnage acquière la tension et le courant du circuit de sortie de l'unité de transformateur.

16. Un instrument portable d'ablation de tissus animaux et végétaux basé sur les ultrasons selon la revendication 15, dans lequel l'unité de commande principale comprend en outre une puce auxiliaire U1 et un circuit périphérique de puce auxiliaire ;
la puce U2 de commande principale de l'unité de commande principale envoie une instruction à la puce auxiliaire basée sur le retour de l'unité d'échantillonnage, dans laquelle l'unité d'échantillonnage acquière la tension et le courant du circuit de sortie de l'unité de transformateur ;
la puce auxiliaire U1 reçoit l'instruction de la puce U2 de commande principale et produit les deux deuxièmes signaux PWM avec cycles de service complémentaire à l'unité de pilotage, dans laquelle les deux deuxièmes signaux PWM avec cycles de service complémentaire sont en outre retournés à la puce U2 de commande principale.

17. Un instrument portable d'ablation de tissus animaux et végétaux basé sur les ultrasons selon la revendication 15, dans lequel les deux deuxièmes signaux PWM avec cycles de service complémentaire sont en outre amplifiés par un circuit de sortie d'amplification de premier signal et un circuit de sortie d'amplification de deuxième signal respectivement puis envoyés à l'unité de pilotage.

18. Un instrument portable d'ablation de tissus animaux et végétaux basé sur les ultrasons selon la revendication 15, comprenant en outre une unité d'affichage, un bouton (6), une interface de chargement, une mémoire, une unité d'interface USB et/ou une unité tactile raccordées à la puce U2 de commande principale ; dans laquelle la puce U2 de commande principale est raccordée au bouton et à l'unité d'affichage, le bouton (6) et un écran d'affichage (7) de l'unité d'affichage sont agencés sur une surface de la deuxième section du logement (1).
